# EUROPÄISCHE PATENTSCHRIFT

(11) **EP 2 711 697 B1**
(45) Veröffentlichungstag und Bekanntmachung des Hinweises auf die Patenterteilung: **25.08.2021**
(21) Anmeldenummer: 13004588.3
(22) Anmeldetag: 19.09.2013
(51) Int. Cl.: G01N 27/02, G01N 33/18, D06F 34/22

(54) **Verfahren und Vorrichtung zur Erfassung von Eigenschaften fluider Medien**
Method and device for measuring characteristics of fluid media
Procédé et dispositif d'enregistrement des propriétés de fluides

(30) Priorität: 19.09.2012 DE 102012018539
(43) Veröffentlichungstag der Anmeldung: 26.03.2014
(73) Patentinhaber: AST (Advanced Sensor Technologies) International Asset GmbH, 75365 Calw-Hirsau (DE)
(72) Erfinder: Gruden, Roman, 75180 Pforzheim (DE); Beck, Volker, 75365 Calw (DE)
(74) Vertreter: Winter, Brandl - Partnerschaft mbB

(56) Entgegenhaltungen:
- DE-A1- 4 311 064
- DE-U1-202011 101 482
- US-A1- 2005 179 449
- ROMAN GRUDEN ET AL: "Influence of surface effects on the characteristic curves of detergent sensors", 2012 9TH INTERNATIONAL MULTI-CONFERENCE ON SYSTEMS, SIGNALS AND DEVICES (SSD 2012) : CHEMNITZ, GERMANY, 20 - 23 MARCH 2012, IEEE, PISCATAWAY, NJ, 20. März 2012 (2012-03-20), Seiten 1-6, XP032180278, DOI: 10.1109/SSD.2012.6198053 ISBN: 978-1-4673-1590-6
- KURZWEIL P ET AL: "A new monitoring method for electrochemical aggregates by impedance spectroscopy", JOURNAL OF POWER SOURCES, ELSEVIER SA, CH, Bd. 127, Nr. 1-2, 10. März 2004 (2004-03-10), Seiten 331-340, XP004494995, ISSN: 0378-7753, DOI: 10.1016/J.JPOWSOUR.2003.09.030
- HELINANDO P. DE OLIVEIRA ET AL: "Use of Electrical Impedance Spectroscopy as a Practical Method of Investigating the Formation of Aggregates in Aqueous Solutions of Dyes and Surfactants", THE JOURNAL OF PHYSICAL CHEMISTRY B, Bd. 115, Nr. 21, 2. Juni 2011 (2011-06-02) , Seiten 6903-6908, XP055067294, ISSN: 1520-6106, DOI: 10.1021/jp200644y

## Beschreibung

Die vorliegende Erfindung betrifft ein Verfahren und eine Vorrichtung zur Erfassung von Eigenschaften fluider Medien, und insbesondere ein Verfahren und eine Vorrichtung zur Erfassung der Eigenschaften wässriger Medien in Verbindung mit der Impedanz-Spektroskopie.

In privaten Haushalten und in industriellen Anwendungen besteht im Allgemeinen ein Informationsbedarf hinsichtlich der Eigenschaften fluider Medien. Fluide Medien können hierbei sämtliche fluide Betriebsstoffe sein, wie wässrige Medien in Form wässriger Lösungen von chemischen Substanzen, sowie Öle und dergleichen. Die Erfassung der Eigenschaften der jeweiligen fluiden Medien ist erforderlich zur Bestimmung grundlegender Eigenschaften bei dem Einsatz dieser Medien, die Bestimmung von im Betrieb auftretenden Veränderungen sowie speziell das Auftreten von Verschmutzungen durch Fremdstoffe in einem bestimmten fluiden Medium. Entsprechend den erfassten Messwerten und den daraus durch Auswertung gewonnenen Erkenntnissen kann entschieden werden, ob das fluide Medium im derzeitigen Zustand noch vorbestimmte Anforderungen erfüllt oder ob Maßnahmen zur Reinigung oder zu einem Austausch des fluiden Mediums erforderlich werden.

Ein anschauliches Beispiel eines fluiden Mediums und insbesondere einer wässrigen Lösung ist die in einem Waschgerät (Waschmaschine, Wascheinrichtung) aufbereitete Waschlauge zur Reinigung von Bekleidung oder anderen Gegenständen. Im Hinblick auf Untersuchungen an einer Waschlauge werden nachstehend bekannte Verfahren und Vorrichtungen genannt.

Die Druckschrift DE 217 557 A1 offenbart ein Verfahren und eine zugehörige Vorrichtung zur Regelung der Reinigungs- oder Spülmittelzugabe in Waschgeräten, wobei in dem Waschgerät zur physikalisch-chemischen Bestimmung der Waschlauge unterschiedliche Sensoren angeordnet sind. Über eine der Auswertung der Ausgangssignale der Sensoren dienenden elektronischen Schaltung wird eine Änderung des Anstiegs eines Mess-Signals während der Zugabe von Reinigungs- oder Spülmitteln erfasst und ausgewertet. In Verbindung mit der Auswertung kann hieraus die Waschmitteldosierung für das Waschgerät gesteuert werden.

Die Druckschrift DE 197 55 418 A1 offenbart ein Sensorelement sowie eine Vorrichtung zur Messung komplexer Impedanzen in Materialien, wobei das Sensorelements zwei Elektroden aufweist, die aus einem leitfähigen Material bestehen und die in einem vorbestimmten Abstand zueinander angeordnet sind. Die beiden Elektroden sind mit einer dünnen Isolierschicht überzogen, die eine im Vergleich zu dem vorbestimmten Abstand geringe Schichtdicke aufweist. Das gebildete Sensorelement ist gegenüber den Umgebungsbedingungen, deren Eigenschaften erfasst werden sollen, weitgehend unempfindlich. In einer Auswerteschaltung werden die Ausgangssignale der Sensoren einer weiteren Verarbeitung unterzogen, und es kann bei dieser Verarbeitung eine Analyse der Eigenschaften einer jeweils zwischen den Elektroden angeordneten Flüssigkeit durchgeführt werden. Hierbei können insbesondere komplexe Impedanzen als Maß für die Eigenschaften der Flüssigkeiten bestimmt und ausgewertet werden.

Im Allgemeinen erfolgt die Reinigung von Bekleidung im privaten und industriellen Bereich weitgehend vollautomatisch mit elektronisch gesteuerten Waschgeräten, die auch als Waschvollautomaten bezeichnet werden. Es ist insgesamt ein Ziel eines Waschablaufs, ein optimales Reinigungsergebnis bei minimalem Einsatz von Wasser, elektrischer und thermischer Energie sowie von Waschmitteln zu erzielen. Moderne Waschgeräte weisen beispielsweise optische Messverfahren zur Erfassung der Trübung einer Waschlauge zur zumindest ungefähren Bestimmung einer Waschmittelkonzentration sowie weitere Verfahren auf, wobei jedoch im Allgemeinen eine aufwendige Datenauswertung erforderlich ist. Trübungssensoren weisen oftmals eine geringe Genauigkeit, wobei das Messergebnis leicht durch Ablagerungen im Bereich des Trübungssensors verfälscht werden kann, die in Waschgeräten in Form von Waschmittel- und Schutzresten häufig auftreten.

Wichtige Größen in Verbindung mit Waschvorgängen sind dabei als grundlegende Größe die Wasserhärte, sowie die Waschmittelkonzentration und die Waschaktivität. Hinsichtlich einer aktuellen Wasserhärte des in ein betreffendes Waschgerät eingebrachten Wassers (im Allgemeinen so genanntes Frischwasser oder Trinkwasser) wird meist von den kommunalen Wasserwerken als zumindest ungefähre Größe bekanntgegeben. In Abhängigkeit von diesen Angaben kann eine Waschmittelmenge nach Abschätzung der Verschmutzung des Waschguts bestimmt werden. Es ist dabei erkennbar, dass hierbei lediglich grobe Abschätzungen vorgenommen werden, mit denen eine tatsächlich genaue Dimensionierung einer optimalen Waschmittelmenge kaum möglich ist.

Eine genaue Bestimmung der so genannten Wasserhärte stellt eine wichtige Grundlage für ein wirtschaftliches und effektives Reinigen von Textilien dar, wobei der Begriff der Wasserhärte sich im Wesentlichen auf die Konzentration der im Wasser gelösten Ionen von Erdalkalimetallen bezieht. Die so genannte Gesamthärte betrifft im Wesentlichen die Kalzium- und Magnesiumionen als gelöste Stoffe im Wasser, sowie auch deren anionische Partner, wie beispielsweise HCO₃⁻. In einer verallgemeinerten Betrachtungsweise betrifft die Erfassung der Eigenschaften eines fluiden Mediums wie Wasser sämtliche im dem Wasser gelöste Stoffe, sowie auch Stoffe, die den Waschvorgang beeinflussen können.

Die Wasserhärte und die damit gelösten Inhaltstoffe sind nachteilig für Waschvorgänge und für Verarbeitungen, bei denen das Wasser erwärmt wird.

Bei kommunalen Wasserwerken wird die Wasserhärte, insbesondere die so genannte Gesamthärte, unter Laborbedingungen durch eine quantitative Analyse in Form einer Titration bestimmt. In einer Probelösung mit einem bekannten Stoff in unbekannter Konzentration wird gezielt ein bekannter Stoff mit bekannter Konzentration (so genannte Maßlösung) hinzugefügt, und es wird das Volumen der verbrauchten Maßlösung bestimmt. Auf der Basis des verbrauchten Volumens der Maßlösung kann auf die unbekannte Konzentration eines bestimmten Stoffes in der Probelösung geschlossen werden.

Es ist erkennbar, dass die quantitative Analyse mittels Titration im Wesentlichen Laborbedingungen erfordert, die hinsichtlich der nötigen Apparate und Einrichtungen aufwendig sind, und es ist des Weiteren erforderlich, eine in ihrer Konzentration genau bestimmte Maßlösung der Probelösung hinzuzufügen. Die Anwendung der quantitativen Analyse mittels Titration bleibt somit einem gut ausgerüsteten Labor vorbehalten und ist in dieser Form nicht anwendbar für eine einfache und schnelle Erfassung von Eigenschaften eines fluiden Mediums, wie beispielsweise von Wasser, in allgemeinen industriellen Anwendungen oder in privaten Haushalten.

Da zunehmend die Waschvorgänge und die verwendeten Waschmittel unter Umweltschutzgesichtspunkten betrachtet werden, ist es erforderlich, sowohl eine Wassermenge als auch eine Waschmittelmenge in Abhängigkeit von dem Verschmutzungsgrad eines Waschguts zu dosieren, wobei ebenfalls die Eigenschaften des Wassers (im Allgemeinen Trinkwasser) zu berücksichtigen sind. Eine gezielte und richtige Waschmitteldosierung führt einerseits zu einer Einsparung an Energie und Wasser, sowie andererseits auch zu einer geringeren Belastung des Abwassers. Zu dem Kriterium der Verschmutzung des Waschguts kommt nun das Kriterium der Eigenschaften des Wassers hinzu, da dies bei der Waschmitteldosierung berücksichtigt werden muss.

In diesem Zusammenhang sollte eine Einrichtung zur Erfassung beispielsweise der Eigenschaften fluider Medien, wie beispielsweise des einem Waschgerät zugeführten Wassers oder der Waschlauge einfach aufgebaut und sicher im Betrieb ausgeführt sein. Im Idealfall sollte jedes Waschgerät mit zumindest einer derartigen Einrichtung ausgerüstet werden, so dass in den Waschgeräten eine genaue Bestimmung der erforderlichen Waschmittelmenge möglich ist. Hierbei besteht das Erfordernis eines einfachen und kostengünstigen Aufbaus, vorzugsweise in Form eines Moduls, sowie einer unkomplizierten und damit auch kostengünstigen Montage, da bei der Anwendung derartiger Erfassungseinrichtungen für Waschgeräte mit größeren Stückzahlen zu rechnen ist.

In DE 20 2011 101 482 U1 ist eine Vorrichtung zur Erfassung von Materialeigenschaften eines Mediums offenbart, die eine Messeinrichtung mit einer Ansteuerungseinrichtung und eine Steuerungseinrichtung hat. Die Ansteuerungseinrichtung ist zur Bestimmung eines Verlaufs einer Impedanz Z des Mediums und zur Ausgabe eines Erfassungssignals vorgesehen. Die Steuerungseinrichtung ist zur Auswertung des Erfassungssignals, zur Bestimmung einer Mehrzahl von charakteristischen Punkten des Verlaufs der Impedanz Z und zur Erzeugung eines Ergebnissignals vorgesehen.

De 43 11 064 A1 offenbart ein Verfahren zur Messung einer Schmutzbefrachtung einer Waschflotte. In dem Verfahren ist ein Kondensator vorgesehen, der aus zwei Elektroden und der dazwischen befindlichen Waschflotte gebildet ist. Der Kondensator liefert Messwerte, die dem Imaginärteil einer Impedanz entsprechen und eine Änderung der Impedanz wird bei dem Verfahren ausgewertet.

Der vorliegenden Erfindung liegt daher die Aufgabe zugrunde, ein Verfahren und eine Vorrichtung zur Erfassung von Eigenschaften fluider Medien derart auszugestalten, dass auf einfache Weise mit einem verminderten Aufwand eine genaue und kontinuierliche Messung der Eigenschaften von fluiden Medien gewährleistet ist und die Vorrichtung kostengünstig in Großserie hergestellt werden kann.

Erfindungsgemäß wird diese Aufgabe mit den in den Patentansprüchen angegebenen Merkmalen hinsichtlich eines Verfahrens und einer Vorrichtung gelöst.

Die erfindungsgemäße Vorrichtung umfasst zumindest die Einrichtungen zur Durchführung der jeweiligen Schritte des Verfahrens gemäß einem ersten Ausführungsbeispiel.

Mit dem erfindungsgemäßen Verfahren und der Vorrichtung zur Erfassung der Eigenschafen des fluiden Mediums mit einer Sensoreinrichtung, deren Signale nach einer entsprechenden Ansteuerung in Verbindung mit der Impedanzspektroskopie ausgebildet werden, können die Eigenschaften des fluiden Mediums, und insbesondere die Eigenschaften eines wässrigen Mediums, wie beispielsweise eines Wassers (Frisch-oder Trinkwasser) zur Befüllung einer Wascheinrichtung, auf einfache Weise und mit vergleichsweise großer Genauigkeit bestimmt werden. Auf der Basis dieser Bestimmung, die eine umfassende Betrachtung des Wassers im Hinblick auf den Waschvorgang beeinflussende gelöste Stoffe beinhaltet, kann der Waschvorgang in effizienter Weise beeinflusst werden, indem der Betrieb der Wascheinrichtung, wie beispielsweise die hinzuzufügende Menge an Waschmittel, die Wassermenge und die Anzahl der Spülvorgänge derart beeinflusst werden kann, dass einerseits ein sehr gutes Waschergebnis erzielt werden kann und andererseits der Waschvorgang wirtschaftlich und mit geringster Umweltbelastung durchgeführt werden kann.

Die Ansteuerung der Sensoreinrichtung erfolgt in der Weise, dass der Verlauf einer komplexen Impedanz Z und der entsprechenden Komponenten (Realteil Re(Z) und Imaginärteil (Im(Z)) der komplexen Impedanz des Mediums entsprechend einem vorbestimmten Frequenzbereich in Abhängigkeit von der Frequenz bestimmt wird. Diese Impedanzkennlinie dient als eine Anfangs-Impedanzkennlinie, die zu Beginn eines Ablaufs oder Prozesses erfasst (gemessen) und gespeichert wird. Die Steuerungseinrichtung ist vorgesehen zur Auswertung des Erfassungssignals als Ausgangssignal des Sensors, wobei in Verbindung mit der Auswertung des Erfassungssignals eine Mehrzahl von charakteristischen Punkten des Verlaufs der komplexen Impedanz Z (d. h. der Anfangs-Impedanzkennlinie) bestimmt wird. In Abhängigkeit von einem Vergleich aktuell gemessener charakteristischer Punkte während des Prozesses und der Anfangs- Impedanzkennlinie (die eine Referenzmessung darstellen kann) erhaltener charakteristischer Punkte können die Eigenschaften des fluiden Mediums nach unterschiedlichen Gesichtspunkten bestimmt werden. Insbesondere werden Differenzen zwischen einander entsprechenden charakteristischen Punkten bestimmt als ein Maß für die Eigenschaften des fluiden Mediums.

Mit der erfindungsgemäßen Lösung ist es möglich, unterschiedliche Eigenschaften von fluiden Medien zu bestimmen, wie beispielsweise eine Waschmittelkonzentration in einer Waschlauge oder auch einen Härtegrad in Frischwasser, so dass seitens eines Verbrauchers Maßnahmen ergriffen werden können, um in Abhängigkeit von den erfassten Eigenschaftswerten optimale Ergebnisse auch im Hinblick auf Umweltschonung zu erzielen. Das Verfahren und die Vorrichtung zur Erfassung der Eigenschaften fluider Medien gemäß der vorliegenden Erfindung ermöglicht somit eine genaue und kontinuierliche Erfassung auf eine einfache Weise, wobei die Sensoreinrichtung dem Medium ausgesetzt ist und die Erfassung mittels einer entsprechenden Ansteuerung über die Steuerungseinrichtung durchgeführt wird und die Ansteuerung in Verbindung mit der Impedanzspektroskopie erfolgt. Hierbei eine Mehrzahl charakteristischer Punkte bestimmt, die mit entsprechenden gespeicherten Grunddaten verglichen werden können.

Es ist daher möglich, dem Benutzer eines Haushaltsgeräts oder auch bei einer industriellen Anwendung, schnell eine gewünschte Information zur Verfügung zu stellen, oder die Information gezielt weiteren Steuerungs- oder Regelungseinheiten oder Stellorganen zuzuführen, so dass Maßnahmen zur Optimierung eines Prozesses durchgeführt werden können. Dies betrifft beispielsweise die Dosierung von Waschmitteln im Wasser mit einer bestimmten Wasserhärte. Die Auswertung sei im Einzelnen auf die Umgebung der charakteristischen Punkte beschränkt, so dass auf diese Weise eine erhebliche Verminderung der Daten möglich ist und damit die Auswertungszeiten vermindert werden. In Verbindung mit einer einfachen Sensoranordnung eröffnet dies die Möglichkeit, die Vorrichtung und das Verfahren gemäß der vorliegenden Erfindung in einem automatisierten System bei industrieller Anwendung und auch in Haushaltsgeräten einzusetzen, wobei der jeweilige Benutzer keine zusätzlichen Maßnahmen oder eine weitere Schulung für die Benutzung der jeweiligen Geräte benötigt.

In Abhängigkeit von dem bestimmten Medium, beispielsweise bei der Anwendung der Impedanzspektroskopie zur Bestimmung der umfassenden Wasserhärte bzw. des Wasserwerts, werden in Verbindung mit der vorab definierten oder erfassten Anfangs-Impedanzkennlinie für dieses Medium die charakteristischen Punkte bestimmt und gemäß der Lage dieser charakteristischen Punkte der Anfangs-Impedanzkennlinie Umgebungs-Frequenzbereiche um diese Punkte definiert. Innerhalb der Umgebungs-Frequenzbereiche werden die aktuellen Messungen durchgeführt, wobei mit dieser Messung eine neue Lage (in Form von Frequenzwerten) des betreffenden charakteristischen Punkts bestimmt wird. Aus der hinsichtlich einer Frequenzinformation bestimmten neuen Lage des betreffenden charakteristischen Punkts aus der aktuellen Messung relativ zur früheren Messung der Anfangs-Impedanzkennlinie innerhalb eines jeweiligen Umgebungs-Frequenzbereichs wird ein Ergebnissignal hinsichtlich der Eigenschaften des Mediums gewonnen. Insbesondere kann im vorliegenden Fall bei der beschriebenen Anwendung der Wasserwert als umfassende und erheblich über die bisher bekannte Gesamthärte eines Frischwassers als spezielle und aktuelle Eigenschaft des Wassers bestimmt werden.

Weitere Ausgestaltungen der Erfindung sind in den Unteransprüchen angegeben.

Es kann der Referenzbereich in Abhängigkeit von dem Anfangswert der Eigenschaften des fluiden Mediums bestimmt werden, und es kann der Anfangswert der Eigenschaften innerhalb des Referenzbereichs liegen.

Der Schritt des Bestimmens der Differenz zwischen den charakteristischen Punkten kann den Schritt des Bestimmens der Differenz zwischen jeweils korrespondierenden charakteristischen Punkten innerhalb des jeweiligen Umgebungs-Frequenzbereichs umfassen.

Der Schritt des Durchführens weiterer Erfassungen kann den Schritt des Bestimmens der aktuellen Impedanzkennlinie innerhalb der Umgebungs-Frequenzbereiche der jeweiligen charakteristischen Punkte umfasst.

Der Schritt des Bestimmens der Differenz zwischen den jeweiligen charakteristischen Punkt kann den Schritt des Bestimmens der Differenz individuell für jeden der charakteristischen Punkte oder für alle verwendeten charakteristischen Punkte umfassen.

Der Referenzbereich der Differenzen zwischen jeweiligen charakteristischen Punkten kann eine obere und eine untere Grenze aufweisen, und die obere und untere Grenze könne entsprechend jeweiliger vorbestimmter Abstände zu dem Anfangswert definiert werden. Es kann dabei die obere und die untere Grenze des Differenzbereichs in Abhängigkeit von der Art des Prozesses bestimmt werden.

Es kann ferner das Verfahren eine Erfassung der Eigenschaften des fluiden Mediums mittels Voltammetrie umfassen, und zur Bildung eines Gesamtergebnisses bezüglich der Eigenschaften des fluiden Mediums können die Erfassungsergebnisse unter Anwendung der Voltammetrie mit den Erfassungsergebnissen unter Anwendung der Impedanzspektroskopie korreliert werden, so dass ein sehr genaues und umfassendes Gesamtergebnis erzielt wird.

In der Vorrichtung kann eine Steuerungseinrichtung vorgesehen sein, die charakteristischen Punkte bei vorbestimmten Frequenzen innerhalb des Umgebungs-Frequenzbereichs zu bestimmen. Ferner kann die Vorrichtung eine Sensoreinrichtung aufweisen zur Durchführung von Erfassungen bezüglich des fluiden Mediums, und es kann die Steuerungseinrichtung des Weiteren vorgesehen sein, die Sensoreinrichtung zur Durchführung von Erfassungen mittels der Impedanzspektroskopie oder der Voltammetrie anzusteuern.

Bei dem Verfahren und der Vorrichtung gemäß einem zweiten Ausführungsbeispiel umfasst die Steuerungseinrichtung zusätzlich zu den Einrichtungen und Möglichkeiten des ersten Ausführungsbeispiels und somit neben der Möglichkeit der Durchführung beliebiger Erfassungen mittels der Impedanz-Spektroskopie (Erfassen von Impedanzkennlinien) die Möglichkeit, Messungen oder Erfassungen gemäß dem Verfahren der zyklischen Voltammetrie (cyclic voltammetry, CV) durchzuführen. Das Verfahren gemäß der zyklischen Voltammetrie kann unabhängig von der Impedanzspektroskopie durchgeführt werden, und es kann die Steuerungseinrichtung die Durchführung einer Erfassung mittels Voltammetrie mit derselben Sensoreinrichtung veranlassen. Jeweilige Erfassungsergebnisse können miteinander korreliert werden zur Bestimmung eines genauen Gesamtergebnisses hinsichtlich der Eigenschaften des fluiden Mediums.

Die Erfindung wird nachstehend anhand von Ausführungsbeispielen unter Bezugnahme auf die Zeichnung näher beschrieben. Es zeigen:
Fig. lein Blockschaltbild einer Schaltungsanordnung (Vorrichtung) zur Durchführung einer Impedanzspektroskopie,
Fig. 2 eine vereinfachte schematische Darstellung der Vorrichtung gemäß Fig. 1, die beispielsweise an einem Gerät angeordnet ist,
Fig. 3 eine Kennlinie der Impedanz Z mit der Darstellung des Imaginärteils über dem Realteil und charakteristischer Punkte P1 bis P4,
Fig. 4 eine grafische Darstellung zur Veranschaulichung von Differenzen aus dem Vergleich entsprechende charakteristischer Punkte unterschiedlicher Erfassungen, und
Fig. 5 eine grafische Darstellung zur Veranschaulichung einer Anwendung der Voltammetrie.

### Beschreibung bevorzugter Ausführungsbeispiele

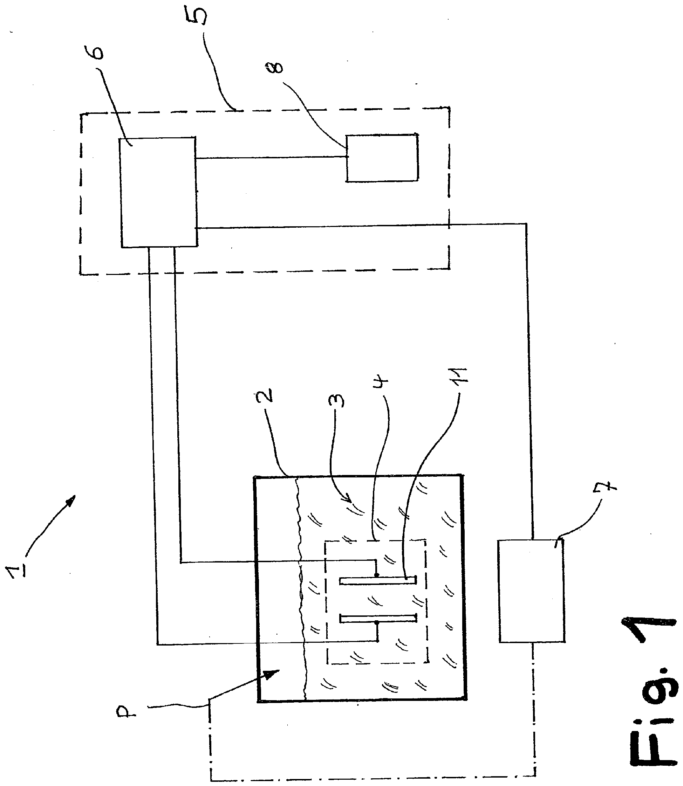

Nachstehend werden anhand des in Fig. 1 gezeigten schematischen Blockschaltbilds der Aufbau und die Wirkungsweise des erfindungsgemäßen Verfahrens sowie der erfindungsgemäßen Vorrichtung gemäß einem ersten Ausführungsbeispiel beschrieben.

Fig. 1 zeigt die Vorrichtung 1 zur Erfassung der Eigenschaften fluider Medien (nachstehend vereinfacht als "die Vorrichtung 1" bezeichnet), wobei die Vorrichtung 1 in einem Behälter 2 mit Kontakt zu einem dort befindlichen fluiden Medium 3 angeordnet ist. Der Begriff des Behälters ist hierbei in sehr allgemeiner Form aufzufassen, wobei es sich beispielsweise um einen Tank in einer Maschine, eine Trommel einer Wascheinrichtung (Waschmaschine) oder einen beliebigen Behälter mit Frischwasser handeln kann. Die vorliegende Erfindung ist nicht auf eine dieser Behälterarten beschränkt. In jedem Fall beinhaltet der Behälter 2 das zu erfassende fluide Medium, beispielweise ein Frischwasser aus einer kommunalen Wasserversorgungseinrichtung, in einer ausreichenden Menge, so dass es mittels einer Sensoreinrichtung 4 hinsichtlich seiner Eigenschaften erfasst werden kann. Die Sensoreinrichtung 4 ist daher ganz oder zumindest teilweise von dem Medium 3 umgeben, wobei die Sensoreinrichtung 4 so weit von dem Medium umgeben sein muss, dass eine ausreichend verlässliche Messung möglich ist. Hierbei ist der Begriff des Mediums ebenfalls sehr allgemein auszulegen, da jedes fluide Medium, wie beispielsweise wässrige Lösungen, Waschlaugen, Öle verschiedener Art und dergleichen einer Messung unterzogen werden können. Zur Vereinfachung der Darstellung wird nachstehend ohne eine Beschränkung hierauf die vorliegende Erfindung in Verbindung mit der Überprüfung von Wasser oder wässrigen Lösungen beschrieben.

Die Vorrichtung 1 gemäß Fig. 1 umfasst ferner eine Steuerungseinrichtung 5, die einerseits zur Steuerung und/oder Regelung der gesamten Abläufe der Erfassung mittels der Impedanzspektroskopie dient, und andererseits eine Ansteuerung der Sensoreinrichtung 4 veranlasst, sowie eine Auswertung der von der Sensoreinrichtung 4 abgegebenen Signale (Erfassungssignale) durchführt.

Zu diesem Zweck umfasst die Steuerungseinrichtung 5 eine Ansteuerungseinrichtung 6, die mit der Sensoreinrichtung 4 verbunden ist und die, entsprechend innerhalb der Steuerungseinrichtung 5 erzeugten Anweisungen und Befehlen, die Sensoreinrichtung 4 mit jeweiligen elektrischen Signalen ansteuert. Beispielsweise werden an die Sensoreinrichtung 4 zur Durchführung entsprechender Messungen Ströme und Spannungen in Verbindung mit vorbestimmten Frequenzen oder Frequenzbereichen angelegt. Die Steuerungseinrichtung 5 kann dabei auch vorgesehen sein zur Erfassung der Temperatur des Mediums 3, und kann hierzu mit einer - in den Figuren nicht gezeigten - Temperaturerfassungseinrichtung verbunden sein. Alternativ kann die Ansteuerungseinrichtung 6 auch außerhalb der Steuerungseinrichtung 5 als separate Einheit angeordnet sein, wobei die Sensoreinrichtung 4 und die Ansteuerungseinrichtung 6 bei dieser alternativen Anordnung hinsichtlich ihrer Funktion zu einer Messeinrichtung zusammengefasst werden können.

Die Steuerungseinrichtung 5 ist ferner mit einer Steuereinheit 7 verbunden, die direkt in Zusammenhang mit Behälter 2 steht. Wird beispielsweise angenommen, dass der Behälter 2 die Trommel einer Wascheinrichtung (Waschgerät, Waschmaschine) ist oder in Verbindung steht mit einem Frischwasserzulauf der Wascheinrichtung, dann stellt die Steuereinheit die eigentliche Einrichtung zur elektronischen Steuerung der Wascheinrichtung dar. In der Wascheinrichtung werden in der entsprechenden programmierten Weise Wassermenge und Waschmittelzulauf sowie Waschtemperaturen und Zeitverlauf eines Waschvorgangs sowie auch Schleuderzeiten und Schleuderdrehzahlen gesteuert. Die Steuerungseinrichtung 5 kann in Abhängigkeit von Messergebnissen in Verbindung mit der Sensoreinrichtung 4 die Steuereinheit 7 dahingehend beeinflussen, dass, abweichend von einem bestimmten Programm, eine größere oder kleinere Wasser- oder Waschmittelmenge eingesetzt oder beispielsweise auch eine Veränderung der Waschtemperatur vorgenommen werden kann. Somit kann durch die Steuerungseinrichtung 5 in Verbindung mit der Steuereinheit 7 eine variable Steuerung oder auch eine Regelung von Einzelfunktionen der Wascheinrichtung vorgenommen werden. Mittels eines Pfeils P ist gemäß Fig. 1 die Einflussnahme der Steuereinheit 7 auf den Waschvorgang schematisch angedeutet.

Die Steuerungseinrichtung 5 umfasst ferner eine Speichereinrichtung 8, in der zur Durchführung der Erfassung der Materialeigenschaften in Verbindung mit der durchzuführenden Impedanzspektroskopie entsprechende Daten und Programme gespeichert sind, auf die innerhalb der Steuerungseinrichtung 5 zugegriffen werden kann.

Hinsichtlich der Wirkungsweise der in Fig. 1 dargestellten Vorrichtung werden der Sensoreinrichtung 4 entsprechend Anweisungen, die innerhalb der Steuerungseinrichtung 5 in Verbindung mit der Ansteuerungseinrichtung 6 erzeugt werden, vorbestimmte Signale der physikalischen Größen, wie Strom, Spannung und Frequenz, zugeführt, und es werden innerhalb der Steuerungseinrichtung 5 über die Ansteuerungseinrichtung 6 die Erfassungssignale der Sensoreinrichtung 4 aufgenommen und verarbeitet, gegebenenfalls in Verbindung mit aus der Speichereinrichtung 8 ausgelesenen Programmen oder Daten. In der Speichereinrichtung 8 können Grunddaten oder bei früheren Messungen erzeugte Erfassungsdaten gespeichert werden, so dass beispielsweise ein Vergleich aktuell gemessener Daten mit Grunddaten hinsichtlich der Eigenschaften des erfassten fluiden Mediums 3 durchgeführt werden kann. In Abhängigkeit von dem Erfassungsergebnis einerseits und einem Vergleichsergebnis andererseits kann im Falle einer Wascheinrichtung die Steuereinheit 7 angewiesen werden, bestimmte Betriebsparameter (Waschmittelkonzentration, Wassermenge, Temperatur) zu ändern oder beizubehalten.

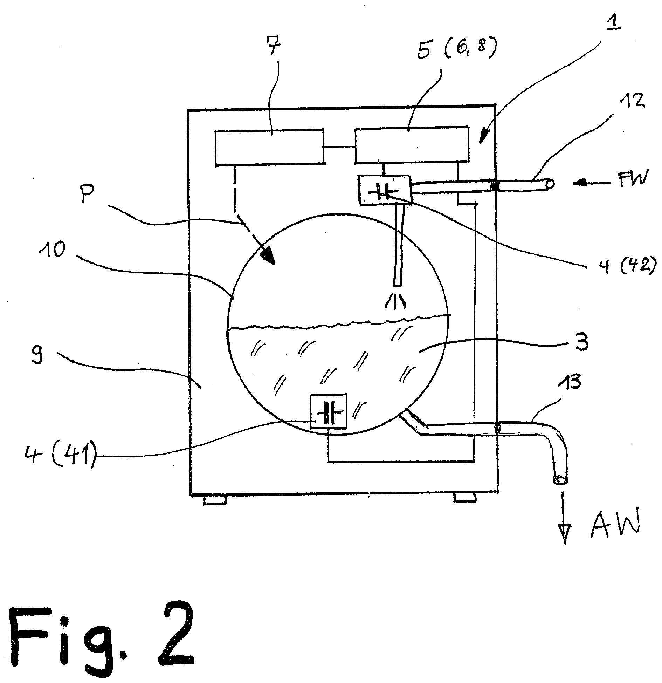

Gemäß Fig. 1 ist die Vorrichtung 1 zur Erfassung der Eigenschaften fluider Medien 3 einschließlich der Sensoreinrichtung 4 und der Steuerungseinrichtung 5 in allgemeiner Form dargestellt. Fig. 2 zeigt demgegenüber die Anordnung der Vorrichtung in einer Wascheinrichtung 9.

In einer Trommel 10 bzw. einem Laugenbehälter der Wascheinrichtung 9 befindet sich das fluide Medium 3, und vorzugsweise in einem tieferen Bereich der Trommel 10 ist die Sensoreinrichtung 4 angeordnet und steht mit der Steuerungseinrichtung 5 in Verbindung, die die Ansteuerungseinrichtung 6 sowie die Speichereinrichtung 8 umfasst. Die Steuerungseinrichtung 5 ist mit der Steuereinheit 7 der Wascheinrichtung 9 verbunden, und mittels eines Pfeils P ist in Fig. 2 in gleicher Weise wie in Fig. 1 veranschaulicht, dass die Steuereinheit 7 der Wascheinrichtung 9 entsprechende Einflüsse auf den Waschvorgang ausübt.

Gemäß Fig. 2 ist im unteren Teil der Trommel 10 oder auch eines in der Figur nicht gezeigten Laugenbehälters, in dem die Trommel 10 drehbar gelagert ist, die Sensoreinrichtung 4 angeordnet. Eine gleichartige oder ähnliche Sensoreinrichtung 4 ist ebenfalls vorzugsweise oberhalb der Trommel 10 in Verbindung mit einem Zulaufrohr 12 für Frischwasser FW angeordnet. Die Sensoreinrichtung 4 umfasst somit gemäß der Darstellung in Fig. 2 einen ersten Sensor 41 im unteren Teil der Trommel 10, sowie einen zweiten Sensor 42 in Verbindung mit der Zuleitung 12 für Frischwasser FW. Bevorzugt kann somit der erste Sensor 41 der Sensoreinrichtung 4 die Eigenschaften von Frischwasser kurz nach dem Einleiten in die Wascheinrichtung 9 sowie nach der Zugabe von Waschmittel die Waschlauge als das fluide Medium 3 erfassen, während der zweite Sensor 42 der Sensoreinrichtung 4 vorzugsweise im Frischwasserzulauf angeordnet ist und die Eigenschaften des Frischwassers FW erfasst. Dies betrifft insbesondere die im Wasser immer gelösten Mineralien, so dass damit die Wasserhärte bestimmt werden kann.

Die vorliegende Erfindung umfasst somit gemäß der Darstellung in Fig. 2 beispielhaft zwei Sensoren 41 und 42, die die Sensoreinrichtung 4 bilden. Die Erfindung ist hierauf jedoch nicht festgelegt, vielmehr kann auch lediglich ein Sensor, beispielsweise der erste Sensor 41 im unteren Teil der Trommel 10 angeordnet sein, oder es können mehr als zwei Sensoren verwendet werden. Unmittelbar nach Einlaufen des Frischwassers FW und vor Beginn des Waschvorgangs können die Eigenschaften des Frischwassers FW (des fluiden Mediums 3) und kann insbesondere die Wasserhärte bestimmt werden.

Wie es aus Fig. 2 erkennbar ist, kann die Vorrichtung 1 ohne Weiteres in eine bestehende und handelsübliche Wascheinrichtung 9 eingebaut werden, wobei lediglich die Sensoreinrichtungen 4 und die Steuerungseinrichtung 5 berücksichtigt werden müssen. Neben der Anwendung bei Waschgeräten besteht auch die Möglichkeit, die erfindungsgemäße Anordnung und das dazugehörige Verfahren bei jeder Art fluider Medien anzuwenden. Gemäß Fig. 2 entspricht die Trommel 10 der Wascheinrichtung 9 dem Behälter 2 der Fig. 1. Ist die Sensoreinrichtung 4 in Form des zweiten Sensors 42 ausgebildet, entspricht der Behälter 2 gemäß Fig. 1 dem Zulaufrohr 12 zur Erfassung der Eigenschaften eines zugeführten Frischwassers FW.

In gleicher Weise kann die erfindungsgemäße Vorrichtung und das zugehörige Verfahren auch bei einer Geschirrspülmaschine oder dergleichen angewendet werden, bei denen ein Frischwasserzulauf erforderlich ist und weitere Funktionen des jeweiligen Geräts zumindest teilweise durch die Eigenschaften des Frischwassers FW und speziell durch die Wasserhärte beeinflusst werden. Die vorliegende Erfindung ist auf die Anwendung in einer Wascheinrichtung nicht beschränkt.

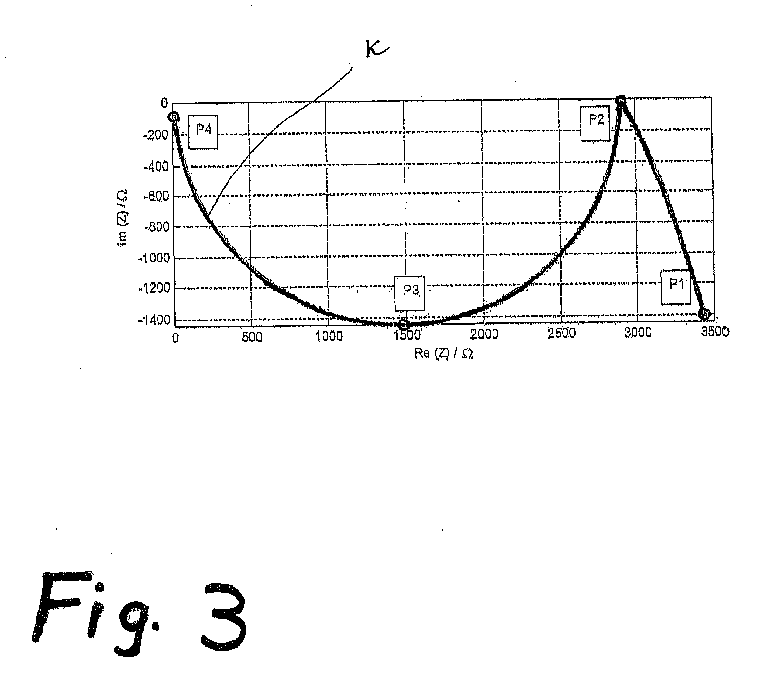

Vorstehend wurde in Verbindung mit den Figuren 1 und 2 der Aufbau und die Wirkungsweise der Vorrichtung 1 beschrieben. In Verbindung einer weiteren Fig. 3 werden Einzelheiten zu dem erfindungsgemäßen Verfahren in Verbindung mit der Impedanzspektroskopie beschrieben.

Wird zumindest einer der Sensoren 41 und 42 der Sensoreinrichtung 4 mittels der Steuerungseinrichtung 5 (Ansteuerungseinrichtung 6) mit entsprechenden elektrischen Signalen zur Durchführung einer Messung angesteuert, ergeben sich für einen vorbestimmten Frequenzbereich von beispielsweise 1 mHz bis 120 MHz Impedanzkennlinien des Realteils und des Imaginärteils der komplexen Impedanz in der Impedanzebene. Fig. 3 zeigt den Verlauf einer Impedanzkennlinie K, wobei die Abszisse den Realteil der Impedanz Z und die Ordinate den Imaginärteil der Impedanz Z zeigt. Aus dem Verlauf der Impedanzkennlinie bzw. der Kennlinie als individuelle und charakteristische Kennlinie für den Messvorgang und das zu erfassende fluide Medium 3 können die speziellen Eigenschaften bestimmt werden. Insbesondere sind Eigenschaften des fluiden Mediums 3 anhand charakteristischer Punkte P1 bis P4 erkennbar, die auf der erfassten Impedanzkennlinie liegen.

Die speziell in Verbindung mit diesen Punkten stehenden Daten sowie ihre Lage im Impedanzspektrum sind von den Eigenschaften des Mediums 3 sowie von den entsprechenden Frequenzen oder Frequenzbereichen der Ansteuerungssignale abhängig. In Verbindung mit der Impedanzspektroskopie sind im Allgemeinen zur Erfassung der Impedanz Z in Verbindung mit einem Sensor zumindest zwei Werte zu erfassen, da es sich bei der Impedanz im Zusammenhang mit den physikalischen Wechselgrößen um eine komplexe Größe handelt, die in einer komplexen Impedanzebene dargestellt werden kann. Es werden hierbei der Realteil Re (Z) und der Imaginärteil Im (Z) der Impedanz oder des Impedanzvektors (gemäß der graphischen Darstellung) bestimmt, woraus die benötigten Werte wie die Impedanz, ihr Betrag, der Phasenwinkel und dergleichen ermittelt werden können.

Bei der Impedanzspektroskopie wird der Impedanzvektor über einen bestimmten Frequenzbereich f (oder Bereich von Winkelgeschwindigkeiten *ω*) zu diskreten Frequenzwerten aufgenommen. Insbesondere wird in Verbindung mit der Impedanzspektroskopie zwischen Strom und Spannung auch der Phasenunterschied zwischen diesen beiden Größen erfasst. Der der Impedanzspektroskopie unterzogene Prüfling ist im Wesentlichen die Sensoreinrichtung 4 (der erste und der zweite Sensor 41 und 42), die eine kapazitive und eine resistive Messung (Leitwertmessung) ermöglicht und die im Wesentlichen in Form eines Plattenkondensators mit Platten 11 (Fig. 1) aufgebaut ist. Zwischen den Platten 11 des Plattenkondensators ist ein elektrisches Feld ausgebildet, das ein Gleichfeld bei Gleichspannungsgrößen oder ein Wechselfeld bei Wechselspannungsgrößen ist. Ein zu prüfender Stoff oder im vorliegenden Fall ein zu prüfendes fluides Medium 3, wie beispielsweise Frischwasser oder eine Waschlauge, sind zwischen den Platten 11 des Plattenkondensators angeordnet, so dass die speziellen dielektrischen Eigenschaften in Verbindung mit der Messung über einen vorbestimmten Frequenzbereich ermittelt werden. In der weiteren Interpretation können auf der Basis der ermittelten dielektrischen Eigenschaften auch weitere Eigenschaften, wie Waschmittelkonzentration in einer Waschlauge oder Fremdstoffkonzentration in einem Frischwasser FW geschlossen werden.

Die einfache Bauform eines Plattenkondensators als Sensoreinrichtung 4 (41, 42) dient zur Veranschaulichung der prinzipiellen Anordnung eines Sensors, wobei weitere Bauformen möglich und für bestimmte Anwendungen zweckmäßig sind. Des Weiteren kann auch bei geeigneten Sensoreinrichtungen eine induktive Messung durchgeführt werden. Auch können mit entsprechenden Sensoreinrichtungen kapazitive und induktive Messungen sowie Leitwertmessungen in Kombination und parallel durchgeführt werden.

Zur Ansteuerung der Sensoreinrichtung 4 kann die Ansteuerungseinrichtung 6 der Steuerungseinrichtung 5 einen Prüf- oder Testsignalgenerator aufweisen, dessen Frequenz beliebig einstellbar ist und beispielsweise innerhalb eines Frequenzbereichs von 1 mHz bis 120 MHz, und beispielsweise von 40 Hz bis 110 MHz.

Wie es aus der graphischen Darstellung von Fig. 3 erkennbar ist, bezeichnen die charakteristischen Punkte P1 bis P4 besondere Positionen innerhalb der erfassten und gemessenen Impedanzkennlinie innerhalb des vorbestimmten Frequenzbereichs zu diskreten Frequenzen, entsprechend dem die Ansteuerungssignale durch die Ansteuerungseinrichtung 6 gebildet wurden. Die Impedanzkennlinie oder Kennlinie K stellt die Endpunkte eines jeweils erfassten Impedanzvektors in der komplexen Impedanzebene dar. Fig. 3 zeigt somit eine graphische Darstellung der komplexwertigen Funktion (der komplexen Impedanz) im Nyquist-Diagramm, wobei die Frequenz der Parameter ist.

Die vorliegende Erfindung ist anhand der beispielsweise vier charakteristischen Punkte P1 bis P4 gemäß Fig. 3 beschrieben, wobei die Erfindung nicht auf diese Anzahl beschränkt ist. Es können weniger oder mehr als vier charakteristische Punkte betrachtet werden. Die charakteristischen Punkte bestehen aus zumindest zwei Punkten oder einer Mehrzahl von Punkten, die in einem eindeutigen Zusammenhang mit den Eigenschaften des fluiden Mediums 3 stehen. Die Erfindung bezieht sich somit in allgemeiner Weise auf eine vorbestimmte Mehrzahln n (n ist eine natürliche Zahl) von charakteristischen Punkten P1 bis Pn. Die Lage der charakteristischen Punkte innerhalb der komplexen Ebene gemäß Fig. 3 ist von den Eigenschaften des fluiden Mediums 3 abhängig. Diese Punkte sind Hoch-Tief- oder Wendepunkte der Kennlinie K (Impedanzkennlinie) deren Lage innerhalb der komplexen Ebene durch die zugehörige Frequenz (oder Winkelgeschwindigkeit *ω*) gekennzeichnet ist. Diese Frequenz wird mittels Durchlaufen eines Teilbereichs eines vorbestimmten Frequenzbereichs sowie durch einen mathematischen Näherungsalgorithmus bestimmt.

Nachstehend wird im Einzelnen das Verfahren zur Erfassung der Eigenschaften des fluiden Mediums 3, wie beispielsweise eines Frischwassers FW in Bezug auf die Wasserhärte, oder in Bezug auf eine Waschlauge im Einzelnen beschrieben.

Die Vorrichtung 1 gemäß der vorliegenden Erfindung ist gemäß Fig. 1 aufgebaut und gemäß Fig. 2 in beispielsweise der Wascheinrichtung 9 angeordnet. Es werden in diesem Zusammenhang die Eigenschaften des Frischwassers FW, die Eigenschaften einer Waschlauge sowie die Eigenschaften eines Abwassers, wie beispielsweise eines aus der Wascheinrichtung 9 mittels eines Ablaufrohrs 13 zu entsorgenden Abwassers AW erfasst. Der Betrieb der Wascheinrichtung 9 sowie die entsprechenden Betriebsparameter können in Abhängigkeit von den Erfassungsergebnissen gesteuert und/oder geregelt werden.

Nach Einleitung eines grundlegenden Messvorgangs in Verbindung mit der Impedanzspektroskopie unter Steuerung durch die Steuerungseinrichtung 5 erfolgt die Bestimmung einer Impedanzkennlinie oder eines Impedanzverlaufs (Kennlinie K gemäß Fig. 3) in Verbindung mit der Vorgabe eines vorbestimmten Frequenzbereichs Δf oder Winkelgeschwindigkeitsbereichs Δ*ω*. Mit den jeweiligen Frequenzsignalen in dem vorbestimmten Frequenzbereich wird die Sensoreinrichtung 4 angesteuert und es wird als Reaktion hierauf die Impedanzkennlinie (Kennlinie K) gemäß Fig. 3 gebildet. Aus der aus der Messung des gesamten Frequenzbereichs hervorgehenden Impedanzkennlinie wird die vorbestimmte Anzahl an charakteristischen Punkten (beispielsweise die charakteristischen Punkte P1 bis P4) bestimmt, und es werden die jeweiligen Impedanzvektoren (Lagepunkte in der komplexen Impedanzebene) für diese charakteristischen Punkte Pn (n = 4) bestimmt.

Es wird hierbei die Anfangs-Impedanzkennlinie vor dem Eigentlichen Beginn eines Prozesses, wie eines Waschvorgangs, bestimmt, wie sie typisch ist für beispielsweise das in die Wascheinrichtung 9 einströmende Frischwasser FW. Das gemessene Ergebnis in Verbindung der damit gebildeten Anfangs-Impedanzkennlinie wird beispielsweise in der Speichereinrichtung 8 gespeichert und kann als Referenzwert dienen. Nachstehend wird auf diese Kennlinie als Anfangs-Impedanzkennlinie Bezug genommen.

Da das von einem kommunalen Wasserversorger bereitgestellte Frischwasser FW (Trinkwasser) natürlichen und auch jahreszeitlich bedingten Schwankungen unterliegt, kann die Wasserhärte zumindest geringen Veränderungen innerhalb von durch Erfahrung gegebenen Toleranzbereichen schwanken. Soll die Waschleistung der Wascheinrichtung 9 hinsichtlich ihrer Wirtschaftlichkeit und Umweltfreundlichkeit verbessert werden, ist es sinnvoll, auch kleine Veränderungen der Wasserhärte des Frischwassers FW vor dem eigentlichen Waschvorgang zu erfassen.

Zu diesem Zweck wird im Rahmen eines vom Benutzer der Wascheinrichtung 9 eingeleiteten Waschvorgangs das Impedanzspektrum des Frischwassers FW aktuell gemessen, und auch diese Werte als Anfangs-Impedanzkennlinie gespeichert. Die Messung betrifft in gleicher Weise wie es im Zusammenhang mit der Anfangs-Impedanzkennlinie beschrieben wurde, die Bildung einer Impedanzkennlinie gemäß Fig. 3, wobei die charakteristischen Punkte, beispielsweise die charakteristischen Punkte P1 bis P4, in der Impedanzkennlinie (Kennlinie K) bestimmt werden. Auf der Basis dieser Anfangs-Impedanzkennlinie und speziell der charakteristischen Punkte, beispielsweise P1 bis P4 gemäß Fig. 3, wird ein Wasserwert Xw erfasst, wobei dieser Wert auch als eine erweiterte Wasserhärte bezeichnet werden kann.

Während die bisherige Wasserhärtebestimmung als sogenannte Gesamthärte die Menge der Kalziumionen und Magnesiumionen betrifft, werden durch die Erfassungsmöglichkeit gemäß der vorliegenden Erfindung in Verbindung mit der Impedanzspektroskopie weitere den Waschprozess beeinflussende Ionen erfasst, wie CU²⁺, Fe²⁺, Fe³⁺, Cl⁻ und SO₄²⁻ sowie Hydrogencarbonat-Ionen HCO₃⁻. Diese weiteren Bestandteile chemischer Verbindungen üben auf den Waschprozess oder Waschablauf ebenfalls einen erheblichen Einfluss aus, so dass für eine sehr genaue Steuerung oder Regelung des Waschprozesses diese weiteren Verbindungen oder Komponenten von Verbindungen berücksichtigt werden müssen. Im Allgemeinen sind die Waschmittel der Hersteller in Sinne eines guten Waschergebnisses auf die Gesamthärte ausgelegt, wobei die Dosierung eines Waschmittels in groben Stufen der bisher bekannten Gesamthärte durch den Benutzer der Wascheinrichtung erfolgt.

Anstelle der üblichen Wasserhärte, die auch als Gesamthärte bezeichnet wird, und die vorzugsweise auf der Basis einer in einem Labor ausgeführten Titration bestimmt wird, wird ein Wasserwert in voll umfänglicher Weise gemäß der vorliegenden Erfindung definiert als ein Wert X_{w}, der von den vorstehend beschriebenen Substanzen oder Komponenten von Substanzen beeinflusst wird. Für den Wasserwert gilt somit

X_{w} = f(Ca²⁺; Mg²⁺; HCO₃⁻; Cu²⁺, Fe²⁺, Fe³⁺, Cl⁻, SO₄²⁻).

Wird dieser Wasserwert als tatsächlicher Erfassungswert einer umfassenden bzw. erweiterten Gesamthärte des Frischwassers FW berücksichtigt, so kann zur Optimierung des Waschprozesses beispielsweise eine Waschmittelmenge, nun auch bezogen auf das verwendete Wasser, individuell dosiert werden. Für einfache Anwendungen oder eine vorläufige Information hinsichtlich der Eigenschaften des fluiden Mediums 3 in Form eines Wassers kann auch eine Teilmenge der erweiterten Wasserhärte bzw. des Wasserwerts herangezogen werden.

Im Zusammenhang mit der Impedanzspektroskopie kann hierbei unter Berücksichtigung der erweiterten Wasserhärte (des Wasserwerts) und der Verschmutzung des Waschguts ein Regelungskonzept gebildet werden zur Bestimmung beispielsweise einer angemessenen Menge eines Waschmittels sowie einer Dauer eines Waschablaufs (Prozess), so dass eine wirkungsvolle Reinigung des Waschguts erreicht wird, während gleichzeitig eine möglichst große Umweltverträglichkeit des Waschablaufs (Prozess) gewährleistet ist.

Neben der Erfassung der aktuellen umfassenden Wasserhärte, die gemäß der vorstehenden Beschreibung als Wasserwert bezeichnet wird, kann auf die gleiche Weise erneut in Verbindung mit der Impedanzspektroskopie und einem gleichartigen Frequenzbereich und somit gleichartiger Ansteuerung der Sensoreinrichtung 4 mittels der Steuerungseinrichtung 5 die Waschlauge der Wascheinrichtung oder auch nach Abschluss des Waschvorgangs das Wasser der einzelnen Spülvorgänge erfasst werden. Relativ zu dem eingangs bestimmten Wasserwert unter Berücksichtigung einer Vielzahl von Einflussfaktoren auf den Waschvorgang kann die Effektivität einzelner Spülvorgänge bestimmt werden. Der eingangs bestimmte Wasserwert Xwo beruht auf der erfassten Anfangs-Impedanzkennlinie.

Es erfolgt somit die Erfassung des Impedanzspektrums des Abwassers AW weiterer Spülvorgänge bei dem Spülen des Waschguts in der Wascheinrichtung 9 mittels der Impedanzspektroskopie, wobei in gleicher Weise eine Impedanzkennlinie K gemäß Fig. 3 vorzugsweise im äquivalenten Frequenzbereich der Ansteuerungssignale für die Sensoreinrichtung 4 erfasst bzw. gebildet wird. Es werden ebenfalls die jeweiligen charakteristischen Punkte in einem ähnlichen Frequenzbereich gebildet. Die verschiedenen Erfassungen während der erforderlichen Spülvorgänge werden als aktuelle Messungen bezeichnet.

Ist beabsichtigt, den Aufwand hinsichtlich der Datenverarbeitung geringer zu halten und die Messleistung (Erfassungen) in der Zeiteinheit zu steigern, werden um die jeweiligen charakteristischen Punkte P1 bis P4 der zuvor gemessenen Anfangs-Impedanzkennlinie des Wasserwerts Frequenzbereiche als Umgebungs-Frequenzbereiche bestimmt, in denen die Messung durchgeführt wird. Hierbei werden die Umgebungsfrequenzbereiche in ungefährer Kenntnis des zu überprüfenden fluiden Mediums 3 bestimmt bzw. in ihrer Lage und in ihrem Frequenzumfang in der komplexen Impedanzebene bestimmt. Auf diese Weise erfolgt eine Beschränkung der Erfassung der aktuellen Eigenschaften des fluiden Mediums 3, beispielsweise der Waschlauge während des Waschvorgangs oder des nach Spülvorgängen zu entsorgenden Abwassers AW, beschränkt auf die Umgebungs-Frequenzbereiche der jeweiligen charakteristischen Punkte, wie beispielsweise der Punkte P1 bis P4 gemäß Fig. 3. Insoweit ist es nicht erforderlich, erneut die gesamte, beispielsweise in Fig. 3 dargestellte Impedanzkennlinie zu erfassen. Vielmehr werden die jeweiligen charakteristischen Punkte (zumindest einer der Punkte P1 bis P4 oder mehrere oder sämtliche) einer neuen aktuellen Impedanz-Kennlinie (d. h. einer aktuellen Messung bzw. Erfassung) innerhalb der vorher bestimmten Umgebungs-Frequenzbereiche bestimmt, so dass lediglich Teile der Impedanz-Kennlinie im Bereich der Umgebungs-Frequenzbereiche verarbeitet werden müssen.

Im Hinblick auf die vorstehend angegebene Abhängigkeit der Lage der charakteristischen Punkte von der Art bzw. den Eigenschaften des fluiden Mediums 3 stehen auch die zugehörigen Umgebungs-Frequenzbereiche in einem Zusammenhang mit dem fluiden Medium 3. Daher werden vorzugsweise die Umgebungs-Frequenzbereiche als Bereich um die jeweiligen charakteristischen punkte (z., B. P1 bis P4) bevorzugt in Kenntnis der Art des zu erfassenden fluiden Mediums bestimmt, sowie unter Berücksichtigung des durchzuführenden Prozesses (wie beispielsweise eines Waschablaufs) und somit voraussichtlich zu erwartender Erfassungsergebnisse (aktuelle Impedanzkennlinien).

Mit jedem Spülvorgang bei einer Wascheinrichtung werden mehr und mehr Rückstände von gelöstem Schmutz und verwendetem Waschmittel aus dem Waschgut ausgespült und entsorgt, so dass das jeweilige Abwasser AW jedes Spülvorgangs im Einzelnen gemäß der vorliegenden Erfindung mittels einer aktuellen Erfassung überprüft wird. Wird bei dieser Überprüfung in den jeweiligen Umgebungs-Frequenzbereichen der charakteristischen Punkte der zu Beginn des Waschvorgangs ermittelten Anfangs-Impedanzkennlinie eine entsprechende Differenz zu den aktuell gemessenen charakteristischen Punkten innerhalb dieser Umgebungs-Frequenzbereiche ermittelt, ist diese Differenz ein Maß für die Veränderung der Eigenschaften des verwendeten Wassers vom eingangs zugeführten Frischwasser FW zum nach einem Spülvorgang zu entsorgenden Abwasser AW.

Im Einzelnen tritt hierbei ein Unterschied auf zwischen dem eingangs ermittelten charakteristischen Punkt, beispielsweise P2, und dem bei den nachfolgenden Messungen des Abwassers gemessenen korrespondierenden charakteristischen Punkt P2 in demselben Umgebungs-Frequenzbereich, so dass infolge der zumindest leichten Verschmutzung des aus dem Spülvorgang erzeugten Schmutzwassers oder Abwassers AW, dies zu geänderten Eigenschaften des Wassers und damit zu einer anderen Lage des Punkts P2 in der komplexen Impedanzebene (Fig. 3) führt. Dies gilt für sämtliche der ermittelten charakteristischen Punkte, beispielsweise die Punkte P1 bis P4 der Fig. 3, wobei die vorliegende Erfindung nicht auf die Anzahl dieser Punkte beschränkt ist. Das Bestimmen der Differenz zwischen den charakteristischen Punkten P1 bis P4 betrifft somit des Bestimmen der Differenz zwischen jeweils korrespondierenden charakteristischen Punkten P1 bis P4 innerhalb des jeweiligen Umgebungs-Frequenzbereichs.

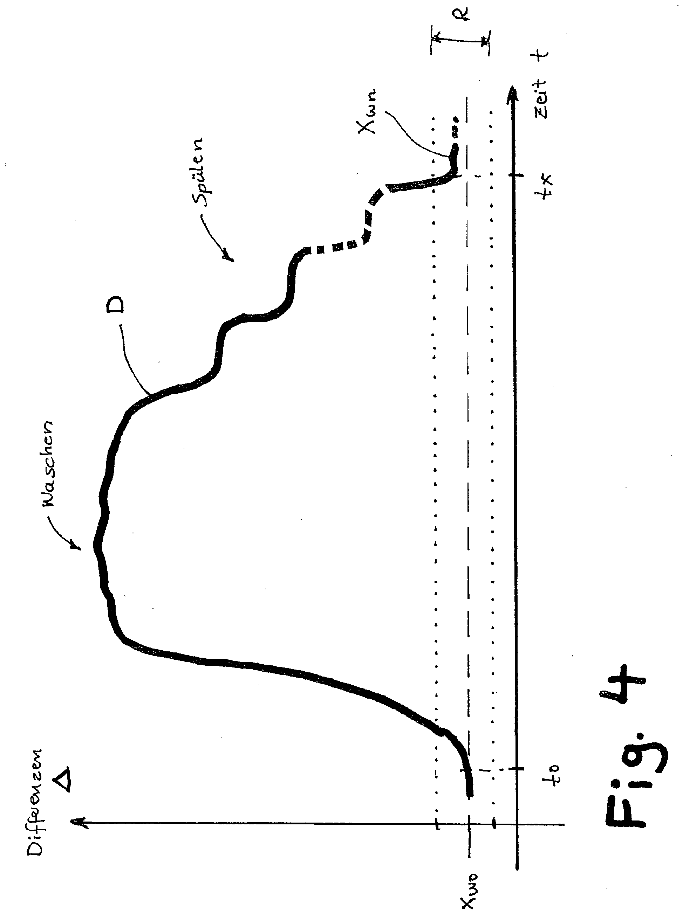

Für jeden der charakteristischen Punkte werden die Differenzen zur eingangs durchgeführten Messung (Anfangs-Impedanzkennlinie) durch aktuelle Erfassungen ermittelt, und es wird zumindest ein Differenzbereich als Referenzbereich R gemäß Fig. 4 definiert, mit dem die jeweils tatsächlich gemessenen Differenzen (Abstände) in Verbindung mit den jeweils gemessenen charakteristischen Punkten P1 bis P4 verglichen werden. In diesem Zusammenhang veranschaulicht Fig. 4 eine Kennlinie D, die einen Verlauf von ermittelten Differenzen in Verbindung mit aktuellen Erfassungen relativ zur Anfangs-Impedanzkennlinie und in Verbindung mit den aktuellen Erfassungen in Umgebungs-Frequenzbereichen der charakteristischen Punkte der Anfangs-Impedanzkennlinie darstellt. Somit umfasst das Bestimmen der Differenz zwischen den charakteristischen Punkten den Schritt des Bestimmens der Differenz zwischen jeweils korrespondierenden charakteristischen Punkten P1 bis P4 innerhalb des jeweiligen Umgebungs-Frequenzbereichs.

Die Kennlinie bzw. Differenzkennlinie D veranschaulicht somit eine zunehmende und wieder abnehmende Verschmutzung (entsprechend sich stetig verändernder Eigenschaften des fluiden Mediums 3) des anfangs erfassten Frischwassers (Wasserwert) im Verlauf des Prozesses, der hier am Beispiel eines Waschvorgangs beschrieben ist. In Fig. 4 bezeichnet die waagrechte Achse die Zeit und die senkrechte die Differenzen Δ zwischen den zu Beginn des Waschvorgangs gemessenen charakteristischen Punkten und den im Verlauf des Waschvorgangs gemessenen charakteristischen Punkte innerhalb des Umgebungs-Frequenzbereichs um die eingangs gemessenen charakteristischen Punkte.

Liegt als Ergebnis dieses Vergleichs die ermittelte Differenz außerhalb des Referenzbereichs R (der innerhalb des Umgebungs-Frequenzbereichs der eingangs gemessenen charakteristischen Punkte P1 bis P4 der komplexen Impedanzebene liegen kann), wird daraus geschlossen, dass das zur Entsorgung anstehende Abwasser AW eines Spülvorgangs noch relativ viel Verschmutzung aufweist, so dass ein weiterer Spülvorgang in vorbestimmter oder zuvor programmierter Weise erforderlich ist. Liegt demgegenüber der Abstand für einen der charakteristischen Punkte P1 bis P4 innerhalb des Referenzbereichs R, wird bestimmt, dass die Verschmutzung des Wassers relativ zu dem eingangs zugeführten Frischwasser ein angemessenes Minimum erreicht hat, so dass auf einen weiteren Spülvorgang verzichtet werden kann. In diesem Fall kann die Steuerungseinrichtung 5 die Steuereinheit 7 der Wascheinrichtung 9 anweisen, keine weiteren Spülvorgänge durchzuführen und, nach einem entsprechenden Schleudern, den Waschvorgang (Prozess) zu beenden.

In Verbindung mit den jeweiligen Messungen des Abwassers AW einzelner Spülvorgänge wird somit über die Bestimmung der sich ständig verändernden Eigenschaften des Wassers im Verlauf des Prozesses mittels der Impedanzspektroskopie bestimmt, ob ein Verschmutzungsgrad innerhalb eines vorbestimmten zulässigen oder gewünschten Bereichs (d. h. dem Referenzbereich R innerhalb des Umgebungsfrequenzbereichs) liegt. In Abhängigkeit von dem Vergleichsergebnis können somit bestimmte Maßnahmen in Bezug auf die Steuerung und/oder Regelung der Wascheinrichtung 9 (des Prozesses) ergriffen werden. Die Erfassungsergebnisse der Anfangserfassung sowie vorzugsweise sämtlicher weitere Erfassungen innerhalb des Prozesses werden in der Speichereinrichtung 8 zur weitere Verarbeitung und Auswertung gespeichert.

Auf diese Weise ist es möglich, die Waschvorgänge der Wascheinrichtung 9 so zu steuern oder zu regeln, dass einerseits ein optimales Reinigungsergebnis und eine sorgfältige Spülung des Waschguts erreicht wird, und andererseits eine höchstmögliche Umweltschonung gewährleistet ist. Mittels der erfindungsgemäßen Anordnung und dem zugehörigen Verfahren wird erreicht, dass in einer handelsüblichen Wascheinrichtung sowohl die Sensorik als auch die Auswertungseinrichtungen, beispielsweise in Form der Steuerungseinrichtung 5, angeordnet werden können, so dass auf einfache und kostengünstige Weise eine moderne und handelsübliche Wascheinrichtung mit derartigen Sensoren ausgestattet oder nachgerüstet werden kann. Mittels der erfindungsgemäßen Anordnung kann somit eine genaue Messung erzielt werden, ohne dass ein aufwendiger Laborbetrieb oder im Laborbetrieb erforderliche Zusatzstoffe, wie Reagenzstoffe, benötigt werden. Für einen Benutzer kann die Erfassung und Auswertung der Eigenschaften des fluiden Mediums, wie beispielsweise des Frischwassers FW und des Abwassers AW relativ zum Frischwasser FW automatisch und ohne manuellen Eingriff erfolgen.

Der Referenzbereich R gemäß Fig. 4 wurde vorstehend als ein einzelner Bereich beschrieben, der für sämtliche verwendete charakteristische Punkte, wie beispielsweise die charakteristischen Punkte P1 bis P4 von Fig. 3 zur Anwendung kommen kann. Jede individuelle Differenz wird relativ zu diesem Referenzbereich R bewertet. Alternativ hierzu kann für jeden der charakteristischen Punkte P1 bis P4 ein individueller Referenzbereich, beispielsweise ein zugehöriger Referenzbereich R1 bis R4 bestimmt werden, wobei als Grundbedingung zu bestimmen ist, wann das zulässige Minimum an Verschmutzung erreicht ist, wenn ein Teil der bei der Abwassermessung erfassten charakteristischen Punkte innerhalb des jeweiligen Referenzbereichs und ein anderer Teil außerhalb des jeweiligen Referenzbereichs liegt. Der Referenzbereich R der Differenzen zwischen jeweiligen charakteristischen Punkten weist eine obere und eine untere Grenze auf. Die obere und untere Grenze sind entsprechend jeweiliger vorbestimmter Abstände zu dem Anfangswert Xwo definiert. Es können ferner die obere und die untere Grenze des Differenzbereichs R in Abhängigkeit von der Art des Prozesses bestimmt werden.

Fig. 4 veranschaulicht somit die Kennlinie D der Erfassungen des eingangs zugeführten Frischwassers sowie der weiteren Abwasserqualitäten der einzelnen Spülvorgänge, und fallweise auch der Eigenschaften der Waschlauge, wobei zum Beenden des gesamten Waschablaufs ermittelt wird, ob die geänderten Eigenschaften des Abwassers gegenüber denjenigen des Frischwassers innerhalb eines vorbestimmten Bereichs R liegen.

Die Differenzkennlinie D gemäß Fig. 4 bezeichnet die aus der gesamten Messung von Beginn des Zulaufs des Frischwassers bis zur letzten Spülwasserentsorgung jeweils erfassten Differenzen der Impedanzkennlinien, und insbesondere der jeweiligen charakteristischen Punkte P1 bis P4. Zum Zeitpunkt t0 strömt Frischwasser FW in die Wascheinrichtung 9, wobei ein Wasserwert als Anfangswasserwert Xwo in Verbindung mit der Anfangs-Impedanzkennlinie bestimmt wird. Der Referenzbereich R liegt symmetrisch oder asymmetrisch um den Anfangswasserwert Xwo.

Zu späteren Zeiten wird Waschmittel zugeführt und es wird der Prozess in Form des Waschvorgangs durchgeführt. Zu weiteren späteren Zeiten erfolgt ein mehrfaches Spülen des Waschguts, wobei kontinuierlich oder in kurzen Zeitabständen zyklisch die Eigenschaften des fluiden Mediums 3, und im vorliegenden Fall der Waschlauge oder des Spülwassers bestimmt werden. Mit jedem Spülvorgang wird die Verschmutzung des zu entsorgenden Wassers geringer, so dass sich die Differenzkennlinie D dem Anfangswasserwert Xwo infolge geringer werdender Differenzen nähert. Liegen beim n-ten Spülvorgang gemäß der aktuell erfassten Impedanzkennlinie die Differenzen in Verbindung mit den charakteristischen Punkten (beispielsweise P1 bis P4) innerhalb des Referenzbereichs R, wobei dies in der Darstellung der Fig. 4 zum Zeitpunkt tx eintritt, kann danach mit den üblichen Maßnahmen des Waschvorgangs der Waschvorgang (der Prozess) beendet werden.

In den Gesamtprogramm, dem der Prozess, beispielsweise der Waschvorgang unterliegt und das in der Steuereinheit 7 gespeichert und verarbeitet wird können beispielsweise Spülvorgänge als ein Standardvorgang gespeichert sein. Der Spülvorgang erfolgt mit festgelegter Länge und mit vorbestimmter Wassermenge. In Verbindung mit dem Erfassungsergebnissen aus der Impedanzspektroskopie können Steuerungs- oder Regelungseingriffe in die Spülvorgänge stattfinden. Entsprechend dem Messergebnis können sie kürzer oder länger ausgeführt werden und/oder es kann die Wassermenge verändert werden. Mit der dynamischen Erfassung, d. h. einer zyklischen Erfassung des fluiden Mediums 3 (Frischwasser, Waschlauge, Spülwasser) können die Spülparameter (Dauer und Wassermenge) im Sinne einer Steuerung oder einer Regelung variiert werden.

Somit wird in Verbindung mit der kontinuierlichen oder zyklischen Messung des Wasserwerts bzw. der Wassereigenschaften einschließlich der Eigenschaften einer Waschlauge in Verbindung mit der Impedanzspektroskopie eine Differenzkennlinie D als zeitlicher Verlauf von Differenzen relativ zum anfangs bestimmten Wasserwert Xwo bestimmt, entspechend der, in Verbindung mit vorbestimmten Schwellenwerten und einem vorbestimmten Referenzbereich R, eine Beeinflussung der Steuerung und/oder der Regelung der Wascheinrichtung 9 und somit des derzeit laufenden Waschvorgangs möglich ist.

Mit der in Fig. 4 gezeigten Differenzkennlinie D, die in Verbindung mit dem kontinuierlich oder zyklisch gemessenen Wasserwert Xw steht, kann eine Differenz-Impedanzkurven-Analyse oder Auswertung durchgeführt werden, wobei lediglich in einem vorbestimmten Bereich der gesamten Impedanzkennlinie, beispielsweise in den Umgebungs-Frequenzbereichen der charakteristischen Punkte P1 bis P4, Messungen bzw. Erfassungen durchgeführt werden und in der Auswertung Abstände oder Differenzen bestimmt werden, die ein Maß für die sich verändernden Eigenschaften beispielsweise des Wassers in der Wascheinrichtung im Verlauf eines Waschvorgangs bilden. Mit der Differenz-Impedanzkurven-Auswertung besteht die Möglichkeit, den Waschvorgang individuell für eine bestimmte Menge an Waschgut, für ein bestimmtes Waschmittel und für bestimmte Grundeigenschaften des zugeführten Frischwassers durchzuführen, so dass gleichzeitig ein gutes Waschergebnis und ein umweltfreundlicher Waschvorgang gewährleistet sind. Mit der Bestimmung des Referenzbereichs, der in Verbindung mit Umweltschutzaspekten hinsichtlich der Verschmutzung des Wassers bestimmt ist, kann erreicht werden, dass nicht unnötig viele Spülvorgänge durchgeführt werden, so dass der Verbrauch an Frischwasser auf das notwendige Maß beschränkt wird und gleichzeitig das Waschgut kaum noch Schmutzreste oder Waschmittelreste aufweist.

Entsprechend dem vorstehend beschriebenen Verfahren und der zugehörigen Vorrichtung (Figuren 1 und 2) umfasst somit die Vorrichtung 1 zumindest sämtliche Einrichtungen, die zur Durchführung des Verfahrens erforderlich sind. Die Steuerungseinrichtung 5, die Ansteuerungseinrichtung 6 sowie die Sensoreinrichtung 4 bilden eine Einrichtung (4, 5, 6) zur Erfassen einer Anfangs-Impedanzkennlinie einschließlich einer Mehrzahl charakteristischer Punkte P1 bis P4 auf der Anfangs-Impedanzkennlinie K in einer komplexen Impedanzebene und Bestimmen eines Anfangswerts Xwo der Eigenschaften des fluiden Mediums 3. Die Steuerungseinrichtung 5und die Speichereinrichtung 8 bilden eine Einrichtung (5, 8) zur Bestimmung jeweiliger Umgebungs-Frequenzbereiche um jeden der charakteristischen Punkte gemäß der Anfangs-Impedanzkennlinie und Durchführen weiterer Erfassungen aktueller Impedanzkennlinien innerhalb der jeweiligen Umgebungs-Frequenzbereiche um die charakteristischen Punkte der Anfangs-Impedanzkennlinie K. Die Steuerungseinrichtung 5 und die Speichereinrichtung 8 bilden eine Einrichtung (5, 8) zur Bestimmung jeweils entsprechender charakteristischer Punkte der aktuellen Erfassungen innerhalb der Umgebungs-Frequenzbereiche der Anfangs-Impedanzkennlinie, und zur Bestimmung jeweils entsprechender charakteristischer Punkte der aktuellen Erfassungen innerhalb der Umgebungs-Frequenzbereiche der Anfangs-Impedanzkennlinie, und die Steuerungseinrichtung 5 und die Speichereinrichtung 8 bilden eine Einrichtung (5, 8) zur Bestimmung von Differenzen zwischen den charakteristischen Punkten jeder der aktuellen Erfassungen der Impedanzkennlinie für zumindest einen der charakteristischen Punkte und dem jeweils entsprechenden charakteristischen Punkt der Anfangs-Impedanzkennlinie, und zum Vergleichen der Differenz mit einem vorbestimmten Referenzbereich R, und zur Durchführung von Steuerungsmaßnahmen bei dem Prozess zu dessen Fortsetzung, wenn die Differenz innerhalb des Referenzbereichs (R) liegt.

Nachstehend wird ein zweites Ausführungsbeispiel der vorliegenden Erfindung beschrieben.

Gemäß dem zweiten Ausführungsbeispiel der vorliegenden Erfindung ist die gleiche Vorrichtung 1 vorgesehen, wie sie im Zusammenhang mit dem ersten Ausführungsbeispiel in den Figuren 1 und 2 dargestellt ist.

Die Vorrichtung 1 im zweiten Ausführungsbeispiel und speziell die Steuerungseinrichtung 5 umfasst zusätzlich zu den Einrichtungen und Möglichkeiten des ersten Ausführungsbeispiels und somit neben der Möglichkeit der Durchführung beliebiger Erfassungen mittels der Impedanz-Spektroskopie (Erfassen von Impedanzkennlinien) die Möglichkeit, Messungen oder Erfassungen gemäß dem Verfahren der zyklischen Voltammetrie (cyclic voltammetry, CV) durchzuführen. Das Verfahren gemäß der zyklischen Voltammetrie (nachstehend als Voltammetrie bezeichnet) kann unabhängig von der Impedanzspektroskopie durchgeführt werden, und es kann die Steuerungseinrichtung 5 und speziell die Ansteuerungseinrichtung 6 zur Durchführung einer Erfassung mittels Voltammetrie dieselbe Sensoreinrichtung 4 mit dem ersten Sensor 41 und dem zweiten Sensor 42 verwenden. Hierbei werden Messungen im Zusammenhang mit der Impedanzspektroskopie und Messungen im Zusammenhang mit der Voltammetrie mit demselben Sensor nacheinander oder intermittierend durchgeführt. Zu einem Zeitpunkt wird ein jeweiliger Sensor durch die Ansteuerungseinrichtung 6 nur in Verbindung mit einem der Erfassungsverfahren angesteuert.

Im Einzelnen verwendet die Voltammetrie eine Dreieckspannung als Ansteuerung der Sensoreinrichtung(en), wobei die Stromantwort über die mit dem fluiden Medium 3 in Kontakt stehende Sensoreinrichtung 4 (41 oder 42) erfasst wird. Die Dreieckspannung liegt im mHz-Bereich und betrifft nur eine Einzelfrequenz oder auch wenige Einzelfrequenzen. Im Gegensatz dazu verwendet das Verfahren der Impedanzspektroskopie im Allgemeinen Spannungen mit sinusförmigem Verlauf im Bereich von etwa 0.1 Hz bis 110 MHz, wobei Erfassungen bei einer sehr großen Anzahl von Einzelfrequenzen erfolgen.

Während die Erfassungsergebnisse der Impedanzspektroskopie im Frequenzbereich dargestellt sind, werden die Erfassungsergebnisse der Voltammetrie im Zeitbereich dargestellt.

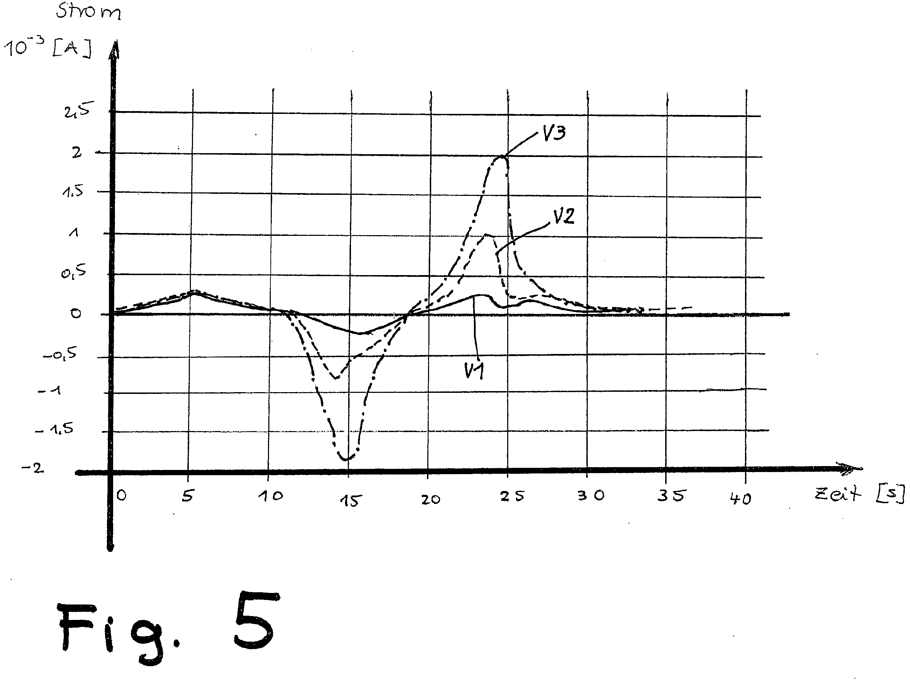

Figur 5 zeigt ein Erfassungsergebnis der zyklischen Voltammetrie, wobei entsprechend einer vorgegebenen Spannung (Dreiecksspannung) die Stromantwort in Abhängigkeit von der Gesamthärte erfasst wird. Die Kennlinie V1 (durchgezogene Linie) zeigt ein Messergebnis eines Wassers mit 2,8 °dH (deutsche Härte). Die Kennlinie V2 (gestrichelte Linie) ergibt sich aus einer Messung eines Wassers mit 8,4 °dH, und die Kennlinie V3 (strichpunktierte Linie) bezeichnet eine Erfassung eines Wassers mit 14° dH. Figur 5 zeigt jeweils den Kennlinienverlauf (Stromantwort) über der Zeit während mehrerer Sekunden. Sie horizontale Achse bezeichnet die Zeit und die vertikale Achse bezeichnet den erfassten Strom.

Die Verfahren der im Impedanzspektroskopie und der Voltammetrie können unabhängig voneinander verwendet werden zur Bestimmung der Eigenschaften des fluiden Mediums 3, wie beispielsweise Wasser mit unterschiedlichen Eigenschaften. Insbesondere kann die Voltammetrie ergänzend zur Impedanzspektroskopie verwendet werden.

In Verbindung mit dem ersten Ausführungsbeispiel wurde eine Erfassung mit dem Verfahren der Impedanzspektroskopie beschrieben, wobei die Messung in der Umgebung zuvor bestimmter charakteristischer Punkte (beispielsweise P1 bis P4) durchgeführt wurde. Gemäß dem zweiten Ausführungsbeispiel kann in Ergänzung dessen eine Messung mittels Voltammetrie durchgeführt werden und es können die aufgenommenen Kennlinien und Daten gespeichert werden (Figur 5). Aus den Erfassungsergebnissen der Impedanzspektroskopie und der Voltammetrie werden die Eigenschaften des fluiden Mediums 3, beispielsweise die Wassereigenschaften und speziell der Wasserwert ermittelt, wobei diese Erfassungen in gleicher Weise wie beim ersten Ausführungsbeispiel jeweilige Anfangskennlinien darstellen. Es kann darauf aufbauend in Verbindung mit dem Prozess am Beispiel eines Waschvorgangs eine Basiswaschmittelmenge aufgrund der ermittelten Wasserdaten aus den Erfassungen mittels der Impedanzspektroskopie und der Voltammetrie bestimmt werden. Es entspricht dies dem Zeitpunkt t0 in Figur 4, zu dem die Anfangs-Impedanzkennlinie und auch die Anfangs-Voltammetriekennlinie gebildet werden und als ein Ausgangspunkt für den anschließenden Prozess dienen.

In dem Prozess am Beispiel des Waschvorgangs wird eine Basiswaschmittelmenge zugegeben und es beginnt der Waschvorgang. Dies ist erkennbar in Figur 4, da sich die Eigenschaften des fluiden Mediums 3, das nun in Form einer Waschlauge vorliegt, erheblich gegenüber den Anfangswerten (Wasserwert Xwo) geändert hat (große Differenzen). Es kann hier, wie beim ersten Ausführungsbeispiel jeweils kontinuierlich oder zyklisch eine Erfassung mittels der Impedanzspektroskopie durchgeführt werden, wobei die erfassten Kennlinien und Daten gespeichert werden. Dies steht wieder im Zusammenhang mit der Bestimmung der charakteristischen Punkte (Lage der charakteristischen Punkte und Umgebungs-Frequenzbereiche). Bei Bedarf können weitere Messungen mittels der Impedanzspektroskopie erfolgen, wobei die charakteristischen Punkte jeweils überwacht werden und mit den anfänglichen Erfassungsergebnissen oder auch mit aktuellen Zwischenergebnissen verglichen werden. Auf diese Weise kann der Verlauf der Eigenschaften der Waschlauge (Figur 4) verfolgt werden. Hinsichtlich entsprechender Steuerungs- oder Regelungsmaßnahmen kann anhand der Erfassungsergebnisse und der Vergleiche (Differenzkennlinie von Figur 4) eine möglicherweise fehlende Waschmittelmenge ermittelt und ergänzt werden.

Ist in dem Prozess der hauptsächliche Waschablauf abgeschlossen und werden Spülvorgänge eingeleitet, dann werden ebenfalls wieder die Messungen mittels der Impedanzspektroskopie durchgeführt, und es werden aktuelle Erfassungsergebnisse mit beispielsweise der Anfangs-Impedanzkennlinie (Anfangswert des Frischwassers Xwo)) verglichen, bis das aktuelle Erfassungsergebnis Differenzen (Abweichungen) zeigt, wie sie in Figur 4 dargestellt sind und schließlich innerhalb des Referenzbereichs R liegen.

Begleitend zu den mehreren Spülvorgängen werden ebenfalls die Voltammetrie-Erfassungsergebnisse aufgezeichnet und mit erfassten Anfangsdaten (Anfangs-Voltammetriekennlinien) verglichen. Unter Berücksichtigung der Erfassungsergebnisse der Impedanzspektroskopie und der Voltammetrie (Korrelation der Ergebnisse) kann auf sehr genaue Weise bestimmt werden, wann die einzelnen Spülvorgänge beendet werden können, da die Erfassungsergebnisse der Wassereigenschaften innerhalb des Referenzbereiches R liegen. Es können in diesem Zusammenhang die jeweiligen Erfassungsergebnisse der Impedanzspektroskopie sowie der Voltammetrie miteinander korreliert, und beispielsweise miteinander verglichen werden.

Mit der Anwendung der Impedanzspektroskopie und der ergänzenden Voltammetrie können die gleichen Vorteile erreicht werden, wie sie in Verbindung mit dem ersten Ausführungsbeispiel beschrieben sind.

Vorstehend wurde die Erfindung und ihre Wirkungswiese anhand eines Waschvorgangs in einer Wascheinrichtung (Waschmaschine) beschrieben. Die vorliegende Erfindung ist jedoch in der Anwendung auf Wascheinrichtungen der beschriebenen Arten nicht beschränkt. Vielmehr können weitere ähnliche Prozesse beeinflusst werden, beispielsweise können auch Geschirrspüler damit ausgestattet werden, so dass die Spülvorgänge eines Geschirrspülers in gleichartiger Weise gesteuert werden können. Des Weiteren besteht die Möglichkeit, Wassereigenschaften in verschiedenen Bereichen der Haushalte, der Industrie und auch in der Natur kontinuierlich oder auch zyklisch zu überprüfen, um insbesondere erwünschte und unerwünschte Abweichungen zu vorbestimmten Grundwerten zu bestimmen. Beispielsweise kann bei der Speicherung von Regenwasser ermittelt werden, wann bei Regen das aufgefangene Wasser eine minimale Verschmutzung (beispielsweise durch eine Verschmutzung der Luft und der Auffangfläche) aufweist. Ist die minimal zulässige Verschmutzung erreicht, kann das überprüfte Wasser einem Sammelbehälter zugeleitet werden.

Mit der einfachen Anordnung ohne manuellen Eingriff und mit der Möglichkeit einer digitalen Speicherung und Weiterverarbeitung der gewonnenen Information können in vielen Bereichen der Haushalte in Industrie und Natur die Vorrichtungen in das zugehörige Verfahren eingesetzt werden. Vielfältige Anwendungen sind auch bei kommunalen Wasserversorgungsunternehmen möglich.

Die Erfindung wurde vorstehend in Verbindung mit Ausführungsbeispielen beschrieben. Es ist jedoch für den Fachmann selbstverständlich, dass die Ausgestaltung der vorliegenden Erfindung gemäß den vorstehend beschriebenen Figuren und die angegebenen Bauteile und Komponenten in den Figuren und der Beschreibung sowie die weitere beispielhafte Angaben nicht einschränkend auszulegen sind. Die Erfindung ist auf die angegebene Darstellung in den Figuren, insbesondere auf Dimensionen und Anordnungen nicht beschränkt. Als zur Erfindung gehörig werden sämtliche Ausführungsformen und Varianten angesehen, die unter die beigefügten Patentansprüche fallen.

## Patentansprüche

1. Verfahren zur Erfassung von Eigenschaften fluider Medien innerhalb eines vorbestimmten Prozesses unter Verwendung der Impedanzspektroskopie, mit den Schritten:
a) Erfassen einer Anfangs-Impedanzkennlinie (K) in einer komplexen Impedanzebene über einen vorbestimmten Frequenzbereich zu Beginn des Prozesses,
b) Bestimmen einer Mehrzahl von charakteristischen Punkten (P1 bis P4) zu diskreten Frequenzen auf der Anfangs-Impedanzkennlinie (K) als Maß für die Eigenschaften des fluiden Mediums (3), und Bestimmen eines Anfangswerts (Xwo) der Eigenschaften des fluiden Mediums (3) auf Basis der Anfangs-Impedanzkennlinie (K) und/oder auf Basis der charakteristischen Punkte (P1 bis P4),
c) Bestimmen jeweiliger Umgebungs-Frequenzbereiche um jeden der charakteristischen Punkte auf der Anfangs-Impedanzkennlinie als jeweiliger Teil der Anfangs-Impedanzkennlinie in Kenntnis der Art des zu erfassenden fluiden Mediums (3),
d) Durchführen weiterer Erfassungen aktueller Impedanzkennlinien innerhalb der und beschränkt auf die jeweiligen Umgebungs-Frequenzbereiche um die charakteristischen Punkte der Anfangs-Impedanzkennlinie (K),
e) Bestimmen jeweils entsprechender charakteristischer Punkte (P1 bis P4) der aktuell erfassten Teile der Impedanzkennlinie innerhalb der Umgebungs-Frequenzbereiche der Anfangs-Impedanzkennlinie,
f) Bestimmen von Differenzen zwischen den charakteristischen Punkten jeder der aktuellen Erfassungen der Impedanzkennlinie für zumindest einen der charakteristischen Punkte innerhalb des betreffenden Umgebungsfrequenzbereichs und dem jeweils entsprechenden charakteristischen Punkt der Anfangs-Impedanzkennlinie, und Vergleichen der Differenz mit einem vorbestimmten Referenzbereich (R), der in Abhängigkeit von dem Anfangswert (Xwo) der Eigenschaften des fluiden Mediums (3) bestimmt wird, und
g) Durchführen von Steuerungsmassnahmen bei dem Prozess zu dessen Fortsetzung, wenn die Differenz innerhalb des Referenzbereichs (R) liegt.

2. Verfahren nach Anspruch 1, wobei der Anfangswert (Xwo) der Eigenschaften innerhalb des Referenzbereichs (R) liegt.

3. Verfahren nach Anspruch 1 oder 2, wobei der Schritt des Bestimmens der Differenz zwischen den charakteristischen Punkten den Schritt des Bestimmens der Differenz zwischen jeweils korrespondierenden charakteristischen Punkten (P1 bis P4) innerhalb des jeweiligen Umgebungs-Frequenzbereichs umfasst.

4. Verfahren nach einem der Ansprüche 1 bis 3, wobei der Schritt des Durchführens weiterer Erfassungen den Schritt des Bestimmens der aktuellen Impedanzkennlinie innerhalb der Umgebungs-Frequenzbereiche der jeweiligen charakteristischen Punkte umfasst.

5. Verfahren nach Anspruch 4, wobei der Schritt des Bestimmens der Differenz zwischen den jeweiligen charakteristischen Punkten den Schritt des Bestimmens der Differenz individuell für jeden der charakteristischen Punkte (P1 bis P4) oder für alle verwendeten charakteristischen Punkte umfasst.

6. Verfahren nach Anspruch 1 oder 2, wobei der Referenzbereich (R) der Differenzen zwischen jeweiligen charakteristischen Punkten eine obere und eine untere Grenze aufweist, und die obere und untere Grenze entsprechend jeweiliger vorbestimmter Abstände zu dem Anfangswert (Xwo) definiert sind und wobei die obere und die untere Grenze des Differenzbereichs (R) in Abhängigkeit von der Art des Prozesses bestimmt wird.

7. Verfahren nach einem der Ansprüche 1 bis 6, wobei das Verfahren eine Erfassung der Eigenschaften des fluiden Mediums (3) mittels Voltammetrie umfasst, und zur Bildung eines Gesamtergebnisses bezüglich der Eigenschaften des fluiden Mediums (3) die Erfassungsergebnisse unter Anwendung der Voltammetrie mit den Erfassungsergebnissen unter Anwendung der Impedanzspektroskopie miteinander korreliert werden.

8. Vorrichtung zur Durchführung des Verfahrens zur Erfassung von Eigenschaften fluider Medien (3) unter Verwendung der Impedanzspektroskopie innerhalb eines vorbestimmten Prozesses, mit:
a) einer Einrichtung, die aus einer Sensoreinrichtung (4), einer Steuerungseinrichtung (5), sowie einer Ansteuerungseinrichtung (6) gebildet ist, zur Erfassung einer Anfangs-Impedanzkennlinie in einer komplexen Impedanzebene über einen vorbestimmten Frequenzbereich zu Beginn des Prozesses,
b) der Einrichtung, die aus der Sensoreinrichtung (4), der Steuerungseinrichtung (5), sowie der Ansteuerungseinrichtung (6) gebildet ist, zur Bestimmung einer Mehrzahl charakteristischer Punkte (P1 bis P4) zu diskreten Frequenzen auf der Anfangs-Impedanzkennlinie (K) als Maß für die Eigenschaften des fluiden Mediums (3), und Bestimmen eines Anfangswerts (Xwo) der Eigenschaften des fluiden Mediums (3) auf Basis der Anfangs-Impedanzkennlinie (K) und/oder auf Basis der charakteristischen Punkte (P1 bis P4),
c) einer Einrichtung, die aus der Steuerungseinrichtung (5) und einer Speichereinrichtung (8) gebildet ist, zur Bestimmung jeweiliger Umgebungs-Frequenzbereiche um jeden der charakteristischen Punkte auf der Anfangs-Impedanzkennlinie als jeweiliger Teil der Anfangs-Impedanzkennlinie in Kenntnis der Art des zu erfassenden fluiden Mediums (3),
d) der Einrichtung, die aus der Steuerungseinrichtung (5) und der Speichereinrichtung (8) gebildet ist, zur Durchführung weiterer Erfassungen aktueller Impedanzkennlinien innerhalb der und beschränkt auf die jeweiligen Umgebungs-Frequenzbereiche um die charakteristischen Punkte der Anfangs-Impedanzkennlinie (K),
e) der Einrichtung, die aus der Steuerungseinrichtung (5) und der Speichereinrichtung (8) gebildet ist, zur Bestimmung jeweils entsprechender charakteristischer Punkte (P1 bis P4) der aktuell erfassten Teile der Impedanzkennlinien innerhalb der Umgebungs-Frequenzbereiche der Anfangs-Impedanzkennlinie,
f) der Einrichtung, die aus der Steuerungseinrichtung (5) und der Speichereinrichtung (8) gebildet ist, zur Bestimmung von Differenzen zwischen den charakteristischen Punkten jeder der aktuellen Erfassungen der Impedanzkennlinie für zumindest einen der charakteristischen Punkte innerhalb des betreffenden Umgebungsfrequenzbereichs und dem jeweils entsprechenden charakteristischen Punkt der Anfangs-Impedanzkennlinie, und zum Vergleichen der Differenz mit einem vorbestimmten Referenzbereich (R), der in Abhängigkeit von dem Anfangswert (Xwo) der Eigenschaften des fluiden Mediums (3) bestimmt wird, und
g) der Einrichtung, die aus der Steuerungseinrichtung (5) und der Speichereinrichtung (8) gebildet ist, zur Durchführung von Steuerungsmassnahmen bei dem Prozess zu dessen Fortsetzung, wenn die Differenz innerhalb des Referenzbereichs (R) liegt.

9. Vorrichtung nach Anspruch 8, wobei die Steuerungseinrichtung (5) vorgesehen ist, die charakteristischen Punkte (P1 bis P4) bei vorbestimmten Frequenzen innerhalb des Umgebungs-Frequenzbereichs zu bestimmen.

10. Vorrichtung nach Anspruch 8 oder 9, wobei die Vorrichtung die Sensoreinrichtung (4) mit einem ersten Sensor (41) und einem zweiten Sensor (42) aufweist zur Durchführung von Erfassungen bezüglich des fluiden Mediums (3) und die Steuerungseinrichtung (5) vorgesehen ist, die Sensoreinrichtung zur Durchführung von Erfassungen mittels der Impedanzspektroskopie oder der Voltammetrie anzusteuern.

## Claims

1. A method for detecting properties of fluid media within a predetermined process using impedance spectroscopy, comprising the steps of:
a) acquiring an initial impedance characteristic (K) in a complex impedance plane over a predetermined frequency range at the beginning of the process,
b) determining a plurality of characteristic points (P1 to P4) at discrete frequencies on the initial impedance characteristic (K) as a measure of the properties of the fluid medium (3), and determining an initial value (Xwo) of the properties of the fluid medium (3) based on the initial impedance characteristic (K) and/or based on the characteristic points (P1 to P4),
c) determining respective ambient frequency ranges around each of the characteristic points on the initial impedance characteristic as a respective part of the initial impedance characteristic with knowledge of the type of the fluid medium (3) to be detected,
d) performing further detections of actual impedance characteristics within and limited to the respective ambient frequency ranges around the characteristic points of the initial impedance characteristic (K),
e) determining respective corresponding characteristic points (P1 to P4) of the actual detected parts of the impedance characteristic within the ambient frequency ranges of the initial impedance characteristic,
f) determining differences between the characteristic points of each of the actual detected portions of the impedance characteristic for at least one of the characteristic points within the concerned ambient frequency range and the respective corresponding characteristic point of the initial impedance characteristic, and comparing the difference with a predetermined reference range (R) determined in dependence on the initial value (Xwo) of the characteristics of the fluid medium (3), and
g) performing control actions on the process to continue the process when the difference is within the reference range (R).

2. The method according to claim 1, wherein the initial value (Xwo) of the properties is within the reference range (R).

3. The method according to claim 1 or 2, wherein the step of determining the difference between the characteristic points comprises the step of determining the difference between respective corresponding characteristic points (P1 to P4) within the respective ambient frequency range.

4. The method according to any one of claims 1 to 3, wherein the step of performing further detections comprises the step of determining the actual impedance characteristic within the ambient frequency ranges of the respective characteristic points.

5. The method according to claim 4, wherein the step of determining the difference between the respective characteristic points comprises the step of determining the difference individually for each of the characteristic points (P1 to P4) or for all used characteristic points.

6. The method according to claim 1 or 2, wherein the reference range (R) of the differences between respective characteristic points has an upper and a lower limit, and the upper and lower limits are defined according to respective predetermined distances from the initial value (Xwo), and wherein the upper and lower limits of the difference range (R) are determined in dependence on the type of the process.

7. The method according to any one of claims 1 to 6, wherein the method comprises detecting the properties of the fluid medium (3) by means of voltammetry, and the detection results using voltammetry are correlated with the detection results using impedance spectroscopy for forming an overall result regarding the properties of the fluid medium (3).

8. An apparatus for carrying out the method for detecting properties of fluid media (3) using impedance spectroscopy within a predetermined process, comprising:
a) a device (4, 5, 6) formed by a sensor device (4), a control device (5), and an activation device (6), for detecting an initial impedance characteristic in a complex impedance plane over a predetermined frequency range at the beginning of the process,
b) the device (4, 5, 6) formed by the sensor device (4), the control device (5), and the activation device (6), for determining a plurality of characteristic points (P1 to P4) at discrete frequencies on the initial impedance characteristic (K) as a measure of the properties of the fluid medium (3), and determining an initial value (Xwo) of the properties of the fluid medium (3) based on the initial impedance characteristic (K) and/or based on the characteristic points (P1 to P4),
c) a device formed by the control device (5) and a storage device (8), for determining respective ambient frequency ranges around each of the characteristic points on the initial impedance characteristic as a respective part of the initial impedance characteristic with knowledge of the type of the fluid medium (3) to be detected,
d) the device formed by the control device (5) and the storage device (8), for performing further detections of actual impedance characteristics within and limited to the respective ambient frequency ranges around the characteristic points of the initial impedance characteristic (K),
e) the device formed by the control device (5) and the storage device (8), for determining respective corresponding characteristic points (P1 to P4) of the actual detected parts of the impedance characteristics within the ambient frequency ranges of the initial impedance characteristic,
f) the device formed by the control device (5) and the storage device (8), for determining differences between the characteristic points of each of the actual detected parts of the impedance characteristics for at least one of the characteristic points within the concerned ambient frequency range and the respective corresponding characteristic point of the initial impedance characteristic, and for comparing the difference with a predetermined reference range (R) determined in dependence on the initial value (Xwo) of the characteristics of the fluid medium (3), and
g) the device formed by the control device (5) and the storage device (8), for performing control actions on the process to continue the process when the difference is within the reference range (R).

9. The apparatus according to claim 8, wherein the control device (5) is provided to determine the characteristic points (P1 to P4) at predetermined frequencies within the ambient frequency range.

10. The apparatus according to claim 8 or 9, wherein the apparatus comprises the sensor device (4) with a first sensor (41) and a second sensor (42) for performing detections with respect to the fluid medium (3) and the control device (5) is provided to control the sensor device to perform detections by means of the impedance spectroscopy or the voltammetry.

## Revendications

1. Procédé pour saisir des propriétés de milieux fluides au sein d'un processus prédéterminé en utilisant la spectroscopie d'impédance, avec les étapes consistant à :
a) saisir une caractéristique d'impédance initiale (K) dans un plan d'impédance complexe sur une plage de fréquence prédéterminée au début du processus,
b) déterminer une pluralité de points caractéristiques (P1 à P4) à des fréquences discrètes sur la caractéristique d'impédance initiale (K) comme une mesure des propriétés du milieu fluide (3), et déterminer une valeur initiale (Xwo) des propriétés du milieu fluide (3) sur la base de la caractéristique d'impédance initiale (K) et/ou sur la base des points caractéristiques (P1 à P4),
c) déterminer des plages de fréquences ambiantes respectives autour de chacun des points caractéristiques sur la caractéristique d'impédance initiale en tant que partie respective de la caractéristique d'impédance initiale en tenant compte de la nature du milieu fluide (3) à saisir,
d) effectuer d'autres saisies des caractéristiques d'impédance actuelles au sein des et limitées aux plages de fréquences ambiantes respectives autour des points caractéristiques de la caractéristique d'impédance initiale (K),
e) déterminer des points caractéristiques correspondants respectifs (P1 à P4) des parties actuellement saisies de la caractéristique d'impédance au sein des plages de fréquences ambiantes de la caractéristique d'impédance initiale,
f) déterminer des différences entre les points caractéristiques de chacune des saisies actuelles de la caractéristique d'impédance pour au moins l'un des points caractéristiques au sein de la plage de fréquence ambiante pertinente et le point caractéristique correspondant respectif de la caractéristique d'impédance initiale, et comparer la différence avec une plage de référence prédéterminée (R) qui est déterminée en fonction de la valeur initiale (Xwo) des propriétés du milieu fluide (3), et
g) effectuer des mesures de commande sur le processus pour le poursuivre si la différence se situe au sein de la plage de référence (R).

2. Procédé selon la revendication 1, dans lequel la valeur initiale (Xwo) des propriétés se situe au sein de la plage de référence (R).

3. Procédé selon la revendication 1 ou 2, dans lequel l'étape de détermination de la différence entre les points caractéristiques comprend l'étape de détermination de la différence entre des points caractéristiques correspondants respectifs (P1 à P4) au sein de la plage de fréquence ambiante respective.

4. Procédé selon l'une quelconque des revendications 1 à 3, dans lequel l'étape consistant à effectuer d'autres saisies comprend l'étape de détermination de la caractéristique d'impédance de courant au sein des plages de fréquences ambiantes des points caractéristiques respectifs.

5. Procédé selon la revendication 4, dans lequel l'étape de détermination de la différence entre les points caractéristiques respectifs comprend l'étape de détermination de la différence individuellement pour chacun des points caractéristiques (P1 à P4) ou pour tous les points caractéristiques utilisés.

6. Procédé selon la revendication 1 ou 2, dans lequel la plage de référence (R) des différences entre des points caractéristiques respectifs présente une limite supérieure et une limite inférieure, et les limites supérieure et inférieure sont définies conformément aux distances prédéterminées respectives par rapport à la valeur initiale (Xwo), et dans lequel les limites supérieure et inférieure de la plage de différences (R) sont déterminées en fonction de la nature du processus.

7. Procédé selon l'une quelconque des revendications 1 à 6, dans lequel le procédé comprend la saisie des propriétés du milieu fluide (3) en utilisant la voltampérométrie, et pour former un résultat global concernant les propriétés du milieu fluide (3), les résultats de saisie en utilisant la voltampérométrie sont corrélés avec les résultats de saisie en utilisant la spectroscopie d'impédance.

8. Dispositif pour effectuer le procédé de saisie des propriétés de milieux fluides (3) en utilisant la spectroscopie d'impédance au sein d'un processus prédéterminé, avec :
a) un appareil, qui est formé à partir d'un appareil de détection (4), d'un appareil de commande (5), ainsi que d'un appareil d'entraînement (6), pour saisir une caractéristique d'impédance initiale dans un plan d'impédance complexe sur une plage de fréquence prédéterminée au début du processus,
b) l'appareil, qui est formé à partir des appareils de détection (4), des appareils de commande (5) et des appareils d'entraînement (6), pour déterminer une pluralité de points caractéristiques (P1 à P4) à des fréquences discrètes sur la caractéristique d'impédance initiale (K) comme mesure des propriétés du milieu fluide (3) et pour déterminer une valeur initiale (Xwo) des propriétés du milieu fluide (3) sur la base de la caractéristique d'impédance initiale (K) et/ou sur la base des points caractéristiques (P1 à P4),
c) un appareil, qui est formé à partir de l'appareil de commande (5) et d'un appareil de mémoire (8), pour déterminer des plages de fréquences ambiantes respectives autour de chacun des points caractéristiques sur la caractéristique d'impédance initiale en tant que partie respective de la caractéristique d'impédance initiale en tenant compte de la nature du milieu fluide (3) à saisir,
d) l'appareil, qui est formé à partir de l'appareil de commande (5) et de l'appareil de mémoire (8) pour effectuer d'autres saisies de caractéristiques d'impédance de courant au sein des et dans les limites des plages de fréquences ambiantes respectives autour des points caractéristiques de la caractéristique d'impédance initiale (K),
e) l'appareil, qui est formé à partir de l'appareil de commande (5) et de l'appareil de mémoire (8), pour déterminer les points caractéristiques correspondants respectifs (P1 à P4) des parties actuellement saisies des caractéristiques d'impédance dans les plages de fréquences ambiantes de la caractéristique d'impédance initiale,
f) l'appareil, qui est formé à partir de l'appareil de commande (5) et de l'appareil de mémoire (8), pour déterminer des différences entre les points caractéristiques de chacune des saisies actuelles de la caractéristique d'impédance pour au moins l'un des points caractéristiques au sein de la plage de fréquence ambiante concernée et le point caractéristique correspondant respectif de la caractéristique d'impédance initiale, et pour comparer la différence avec une plage de référence prédéterminée (R) qui est déterminée en fonction de la valeur initiale (Xwo) des caractéristiques du milieu fluide (3), et
g) l'appareil, qui est formé à partir de l'appareil de commande (5) et de l'appareil de mémoire (8), pour effectuer des mesures de commande sur le processus afin de le poursuivre lorsque la différence se trouve au sein de la plage de référence (R).

9. Dispositif selon la revendication 8, dans lequel l'appareil de commande (5) est prévu pour déterminer les points caractéristiques (P1 à P4) à des fréquences prédéterminées au sein de la plage de fréquence ambiante.

10. Dispositif selon la revendication 8 ou 9, dans lequel le dispositif présente l'appareil de détection (4) avec un premier capteur (41) et un second capteur (42) pour effectuer des saisies par rapport au milieu fluide (3), et l'appareil de commande (5) est prévu pour commander l'appareil de détection pour effectuer des saisies au moyen de la spectroscopie d'impédance ou de la voltampérométrie.
